# EUROPÄISCHE PATENTSCHRIFT

(11) **EP 2 225 293 B1**
(45) Veröffentlichungstag und Bekanntmachung des Hinweises auf die Patenterteilung: **08.02.2012**
(21) Anmeldenummer: 08858613.6
(22) Anmeldetag: 05.12.2008
(51) Int. Cl.: C08F 224/00

(54) **COPOLYMER UND OPHTHALMOLOGISCHE ZUSAMMENSETZUNG**
COPOLYMER AND OPHTHALMOLOGICAL COMPOSITION
COPOLYMÈRE ET COMPOSITION OPHTALMOLOGIQUE

(30) Priorität: 11.12.2007 DE 102007059470; 19.08.2008 DE 102008038390
(43) Veröffentlichungstag der Anmeldung: 08.09.2010
(73) Patentinhaber: Carl Zeiss Meditec AG, 07745 Jena (DE)
(72) Erfinder: RITTER, Helmut, 42111 Wuppertal (DE); SCHMITZ, Daniel, 50677 Köln (DE); NACHBAUR, Jürgen, 13509 Berlin (DE)
(74) Vertreter: Lechner, Armin Anton
(86) Internationale Anmeldenummer: PCT/EP2008/066904
(87) Internationale Veröffentlichungsnummer: WO 2009/074520

(56) Entgegenhaltungen:
- EP-A2- 0 231 572
- EP-A2- 0 383 074
- WO-A1-82/00295
- US-A- 3 721 657
- US-A- 4 082 894
- US-A- 4 948 855

## Beschreibung

### Technisches Gebiet

Die vorliegende Erfindung bezieht sich auf ein Copolymer, welches sich zur Herstellung von ophthalmischen Linsen und insbesondere von Intraokularlinsen und ophthalmischen Implantaten eignet. Die Erfindung betrifft weiterhin eine ophthalmologische Zusammensetzung sowie ihre Verwendung, insbesondere als Augenimplantat (Intraokularlinse).

### Stand der Technik

Es ist bekannt Intraokularlinsen (IOL), welche üblicherweise einen optischen und einen nicht-optischen Teil umfassen, einteilig oder mehrteilig auszubilden. Bei einteiligen Intraokularlinsen bestehen die optischen und die nicht-optischen Teile aus einem einzigen Werkstoff. Bei mehrteiligen IOLs können die optischen und die nicht-optischen Teile aus verschiedenen Werkstoffen bestehen. Die nicht-optischen Teile werden auch als haptische Teile bezeichnet und dienen der Befestigung.

Intraokularlinsen werden in das Auge eingeführt. Um eine Verringerung der Länge des zur Einführung der Intraokularlinse ins Auge erforderlichen Schnittes zu erzielen, ist es wünschenswert, einen geeigneten Werkstoff als Grundmaterial für die IOL, meist ein geeignetes Copolymer zur Verfügung zu stellen, welches eine Verkürzung des Einschnittes z. B. aufgrund seiner Flexibilität und seiner Entfaltungseigenschaften ermöglicht. Somit sollte ein Copolymer, aus dem eine IOL bereitgestellt wird, diesbezügliche vorteilhafte Eigenschaften aufweisen.

In der DE 899 181 08 T2 wird eine einteilige Intraokularlinse mit einem optischen und einem haptischen Teil vorgeschlagen, bei der der optische und der haptische Teil aus demselben Copolymer hergestellt werden. Das Copolymer enthält ein hydrophiles Monomer und ein Alkoxyalkyl-Methacrylat-Monomer, wobei der optische Teil und der haptische Teil in der einteiligen IOL aus demselben Copolymer gebildet sind. Das Copolymer dieser einteiligen Intraokularlinse weist einen Wassergehalt von ca. 10 bis ca. 38 Gewichtsprozent des Gesamtgewichts des hydratisierten Copolymers auf.

In der DE 699 181 08 T2 wird vorgeschlagen, dass zusätzlich zu bereits genannten Monomeren nicht mehr als 10 Gewichtsprozent eines weiteren Monomeren in dem Copolymer enthalten sein können, so dass 90 Gewichtsprozent des trockenen Copolymers auf das hydrophile Monomer in Kombination mit dem Alkoxyalkyl-Methacrylat entfallen.

Das Copolymer der DE 899 181 08 T2 soll eine verbesserte Faltbarkeit aufweisen. Allerdings besteht im Stand der Technik der Bedarf, Copolymere zur Verfügung zu stellen, die eine weiter verbesserte Faltbarkeit und optimierte mechanische Eigenschaften in Kombination mit einer verbesserten Verträglichkeit aufweisen.

Die US 3 721 657 A offenbart ein wasserunlösliches Harz zur Herstellung von Kontaktlinsen, wobei das Harz im Wesentlichen aus Vinylpyrrolidon- und Hydroxyalkylmethacrylat-Monomeren hergestellt wird. Zur Modifizierung des Harzes kann zusätzlich etwa 1 Gew.-% Tetrahydrofurfurylmethacrylat verwendet werden.

Die US 4 948 855 A offenbart Copolymere zur Herstellung von sauerstoff-permeablen Kontaktlinsen. Die Copolymere werden aus ethylenisch ungesättigten Siloxanylalkoxyester-Monomeren, ethylenisch ungesättigten Fluorcarbonester-Monomeren und ethylenisch ungesättigten Sulfonen hergestellt.

Es ist weiterhin bekannt, dass die Netzhaut des Auges gegen phototoxische Einflüsse von Strahlung im Ultraviolettbereich (200 nm bis 400 nm) und violettem des sichtbaren Lichts Bereich (400 nm bis 440 nm) mit Hilfe von molekularen Absorbern geschützt werden kann. Derartige Absorber können im optischen Bereich zur Verwendung in Intraokularlinsen (IOL) vorgesehen sein. Auf dem Markt befindliche Intraokularlinsen absorbieren insbesondere im violetten Lichtbereich nur teilweise. Größenordnungsmäßig treten 25% bis 35% des phototoxischen Lichts mit einer Wellenlänge von 430 nm durch das herkömmliche Linsenmaterial hindurch.

Untersuchungen zeigen, dass der violette Lichtanteil eine entscheidende Rolle bei der Ausprägung einer Altersbedingten Makula-Degeneration (AMD) spielt. Diese beginnt durch Ablagerungen von so genannten Drusen, Stoffwechselendprodukten (Lipofuszinen), und kann in fortgeschrittenem Stadium in einen flächigen Zelltod (geographische Atrophie) des retinalen Pigmentepithels übergehen.

Andererseits ist für die Photorezeption, insbesondere für das Sehen bei verminderten Lichtverhältnissen (scotopic Vision), d.h. beim Dämmerungs- und Nachtsehen, die Durchlässigkeit des Linsenmaterials im blauen Lichtspektrum (etwa 450 nm bis 500 nm) von entschiedener Bedeutung. In diesem blauen Wellenlängenbereich soll so wenig Licht wie möglich absorbiert werden, um eine Beeinträchtigung des Dämmerungs- und Nachtsehens auszuschließen. Auf dem Markt befindliche IOL weisen in diesem Wellenlängenbereich (z.B. bei 475 nm) jedoch eine Transmission von nur etwa 70% bis 75% auf.

### Darstellung der Erfindung

Die Aufgabe der vorliegenden Erfindung ist es somit, eine ophthalmische Linse zur Verfügung zu stellen, die die Nachteile des Standes der Technik überwindet und insbesondere ein vorteilhaftes Eigenschaftsprofil für Intraokularlinsen aufweist.

Die technische Aufgabe der vorliegenden Erfindung wird gemäß einem ersten Aspekt durch eine erfindungsgemäße ophthalmische Linse mit einem Copolymer gelöst, wobei das Copolymer:
a) bezogen auf das Gesamtgewicht des Copolymers 20 bis 95 Gewichtsprozent Struktureinheiten, stammend aus wenigstens einem hydrophilen Monomer, und
b) bezogen auf das Gesamtgewicht des Copolymers 5 bis 80 Gewichtsprozent Struktureinheiten, stammend aus wenigstens einem Monomer gemäß der allgemeinen Formel I 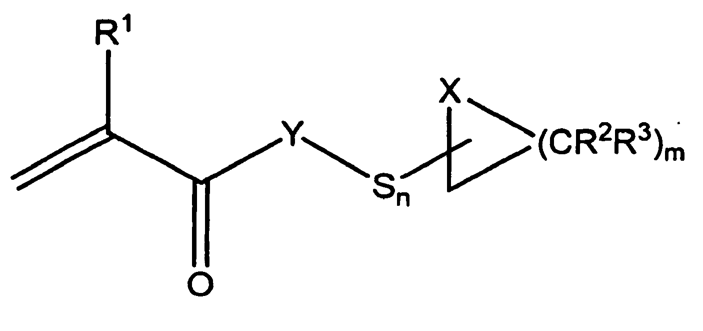
umfasst, wobei
R¹, R² und R³ jeweils unabhängig voneinander Wasserstoff oder Alkyl- bedeuten,
Y: O oder NR⁴ mit R⁴ ausgewählt aus Wasserstoff oder Alkyl- bedeutet,
X: O, S, SO oder SO₂ bedeutet,
S: eine Struktureinheit ausgewählt aus CHR⁵ oder (CHR⁵CHR⁵O)ᵢCH₂ bedeutet, wobei alle R⁵ jeweils unabhängig voneinander Wasserstoff oder Alkyl- bedeuten,
n und i unabhängig voneinander eine ganze Zahl zwischen 1 und 10 und
m eine ganze Zahl zwischen 2 und 6 bedeuten,
und wobei das Copolymer bezogen auf das Gesamtgewicht des Copolymeren nach der Quellung in Wasser einen Wassergehalt von 1 bis 59 Gewichtsprozent aufweist. Alle Stereoisomere und racemischen Gemische der Monomeren a) und b) sind dabei grundsätzlich als mitumfasst anzusehen. Überraschenderweise zeigt das Copolymer der ophthalmischen Linse der vorliegenden Erfindung ein verbessertes Eigenschaftsprofil verglichen mit den Copolymeren des Standes der Technik. Insbesondere weist das Copolymer verbesserte Eigenschaften auf, wenn es in die ophthalmische Linse eingearbeitet wird. Eine solche ophthalmische Linse und insbesondere eine Intraokularlinse lässt sich bei der Implantation besser falten, sodass der chirurgische Eingriff vor Einführung der Intraokularlinse ins Auge einen kleineren Einschnitt verlangt. Außerdem ist die Verträglichkeit eines solchen Copolymers im Auge verbessert. Das Copolymer weist des weiteren eine verbesserte Verarbeitbarkeit zur Herstellung einer ophthalmischen Linse auf. Im Vergleich zu den Copolymeren des Standes der Technik lässt sich das Copolymer der vorliegenden Erfindung in verbesserter Weise mechanisch bearbeiten, um eine Intraokularlinse zu erhalten.

In einer vorteilhaften Ausgestaltung der Erfindung ist vorgesehen, dass das Monomer b) des Copolymers folgende Struktur: 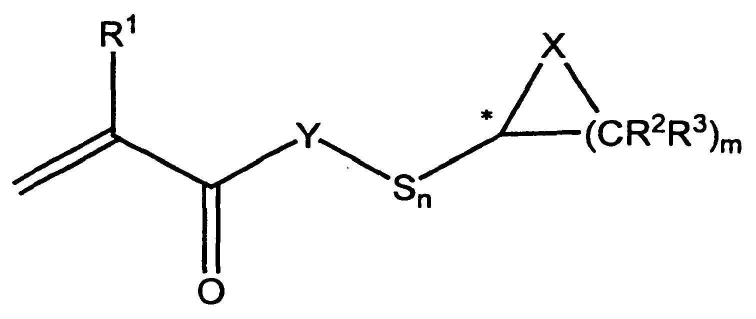 aufweist. Hierdurch weist das Monomer b) neben den vorstehend erläuterten Vorteilen zusätzlich verbesserte Eigenschaften auf, wenn es in eine ophthalmische Linse eingearbeitet wird.

In einer bevorzugten Ausführungsform sind die Reste R¹, R², R³ R⁴ und R⁵ jeweils unabhängig voneinander ausgewählt aus unverzweigten und/oder verzweigten Alkylgruppen mit vorzugsweise 1, 2, 3, 4, 5, 8, 7, 8, 9 und/oder 10 Kohlenstoffatomen. Weiter bevorzugt sind die Reste R¹, R², R³, R⁴ und R⁵ unabhängig voneinander eine Methylgruppe, Ethylgruppe, n-Propylgruppe, iso-Propylgruppe, n-Butylgruppe, iso-Butylgruppe und/oder tert-Butylgruppe.

In einer weiter bevorzugten Ausführungsform ist die Struktureinheit S eine Methylengruppe und in einer weiter bevorzugten Ausführungsform ist die Struktureinheit S eine -CH(CH₃)CH₂OCH₂- Gruppe.

In einer weiter bevorzugten Ausführungsform sind n und i unabhängig voneinander 1, 2, 3, 4, 5, 8, 7, 8, 9 und/oder 10. In einer weiter bevorzugten Ausführungsform ist m 2, 3, 4, 5 oder 6.

In einer weiter bevorzugten Ausführungsform ist der Rest R¹ eine Methylgruppe, wenn die Struktureinheit Y ein O Atom ist. Es ist weiter bevorzugt, dass der Rest R¹ Wasserstoff darstellt, wenn die Gruppe Y NH ist.

Es ist weiter bevorzugt, dass bezogen auf das Gesamtgewicht des Copolymers 30 bis 79 Gewichtsprozent Struktureinheiten aus dem wenigsten einen hydrophilen Monomer a) und weiter bevorzugt 50 bis 79 Gewichtsprozent Struktureinheiten aus dem wenigstens einen hydrophilen Monomer a) in dem Copolymer stammen.

In einer weiter bevorzugten Ausführungsform stammen bezogen auf das Gesamtgewicht des Copolymers in dem Copolymer 10 bis 79 Gewichtsprozent, insbesondere 21 bis 60 Gewichtsprozent, vorzugsweise 21 bis 50 Gewichtsprozent und weiter bevorzugt 21 bis 35 Gewichtsprozent aus dem wenigstens einen Monomer b) gemäß der allgemeinen Formel I. Insbesondere können auch bezogen auf das Gesamtgewicht des Copolymers in dem Copolymer 10 bis 35 Gewichtsprozent aus dem wenigstens einen Monomer b) gemäß der allgemeinen Formel I stammen.

Vorzugsweise weist das Copolymer einen Wassergehalt von 2 bis 50 Gewichtsprozent, weiter bevorzugt von 5 bis 40 Gewichtsprozent und besonders bevorzugt zwischen 10 und 30 Gewichtsprozent bezogen auf das Gesamtgewicht des Copolymers auf.

Die in im Rahmen der Offenbarung angegebenen Anteile an Struktureinheiten stammend aus Monomeren beziehen sich auf das Gesamtgewicht des Copolymers und diese einzelnen Anteile und der Wassergehalt sind bevorzugt so zu wählen, dass in Summe 100 Gewichtsprozent erhalten werden. Für den Fall, dass weitere Inhaltsstoffe in dem Copolymer enthalten sind, sind diese Gewichtsanteile und der Wassergehalt so zu wählen, dass sich ein Gesamtgewicht des Copolymers inklusive der weiteren Inhaltsstoffe zu 100 Gewichtsprozent ergibt.

Es ist weiter bevorzugt, dass das hydrophile Monomer a) ein Monomer der allgemeinen Formel II 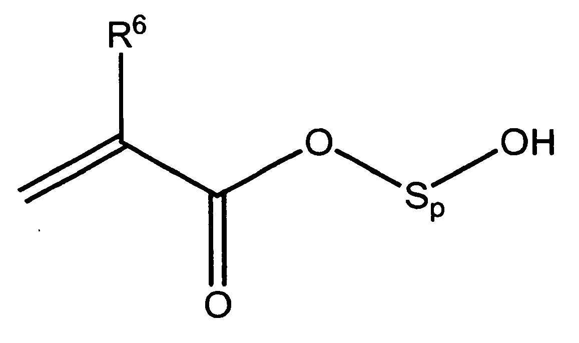 ist, wobei
S: eine Struktureinheit ausgewählt aus CHR⁷ oder (CHR⁷CHR⁷O)ₖCH₂ bedeutet, wobei alle R⁷ jeweils unabhängig voneinander Wasserstoff oder Alkyl- bedeuten, und
p und k unabhängig voneinander eine ganze Zahl zwischen 1 und 10 bedeuten. Auch hierbei sind alle Stereoisomere und racemischen Gemische als mitumfasst anzusehen.

In einer bevorzugten Ausführungsform sind die Reste R⁶ und R⁷ jeweils unabhängig voneinander ausgewählt aus unverzweigten und/oder verzweigten Alkylgruppen mit vorzugsweise 1, 2, 3, 4, 5, 8, 7, 8, 9 und/oder 10 Kohlenstoffatomen. Weiter bevorzugt sind die Reste R⁶ und R⁷ jeweils unabhängig voneinander eine Methylgruppe, Ethylgruppe, n-Propylgruppe, iso-Propylgruppe, n-Butylgruppe, iso-Butylgruppe und/oder tert-Butylgruppe. Für R⁶ ist unabhängig davon besonders bevorzugt, dass R⁶ Methyl- oder H ist.

Für einige Fälle kann es bevorzugt sein, dass das Copolymer keine Struktureinheiten enthält, die aus wenigstens einem Alkoxyalkyl-Methacrylatmonomer und/oder einem Alkoxyalkyl-Acrylatmonomer stammen.

In einer weiter bevorzugten Ausführungsform sind k und p unabhängig voneinander 1, 2, 3, 4, 5, 8, 7, 8, 9 und/oder 10.

In einer noch weiter bevorzugten Ausführungsform ist das hydrophile Monomer der allgemeinen Formel II Hydroxyethylmethacrylat (HEMA) und/oder Hydroxypropylmethacrylat (HPMA). Alternativ oder zusätzlich kann Glycerolmonomethacrylat als hydrophiles Monomer vorgesehen sein.

Es ist weiter bevorzugt, dass das Monomer der allgemeinen Formel I folgende Struktur 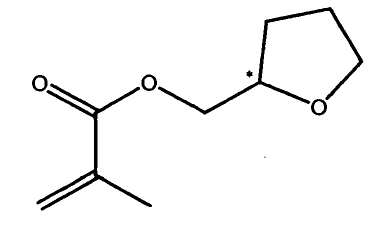 aufweist. Dieses Monomer (Tetrahydrofuran-2-yl)-methylmethacrylat ist dabei auch unter dem Trivialnamen Tetrahydrofurfurylmethacrylat (THFMA) bekannt. Alternativ oder zusätzlich kann hierbei auch das Stellungsisomer (Tetrahydrofuran-3-yl)-methylmethacrylat vorgesehen sein.

In einer bevorzugten Ausführungsform liegen die Monomere der allgemeinen Formel I und/oder II in enantiomerenreiner Form vor. Alternativ bevorzugt können die Monomere der allgemeinen Formel I und/oder II als racemisches Gemisch vorliegen.

In einer weiter bevorzugten Ausführungsform umfasst das Copolymer wenigstens einen oder mehrere Vernetzer. Als geeignete Vernetzer können Vinylmonomere oder -oligomere vorgesehen sein, die zwei oder mehr polymerisierbare Gruppen aufweisen. Hierdurch kann das Copolymer gezielt dreidimensional vernetzt und der Vernetzungsgrad optimal in Abhängigkeit des jeweiligen Anwendungszwecks eingestellt werden. Beispielsweise können als Vernetzer Ethylenglycoldimethacrylat (EGDMA), Trimethylolpropantri(meth)acrylat, 1,3-Glycerindi(meth)acrylat und/oder Butandioldi(meth)acrylat vorgesehen sein.

In einer weiter bevorzugten Ausführungsform enthält das Copolymer einen UV-Absorber. Unter UV-Absorber sind dabei organische oder anorganische Verbindungen zu verstehen, die Strahlung in einem Wellenlängenbereich zwischen 200 nm und 400 nm zumindest überwiegend und vorzugsweise quantitativ absorbieren. Als UV-Absorber ist ein biokompatibles UV-Lichtschutzmittel vorgesehen, wofür Cumarinderivate, die gegebenenfalls über Alkylspacer mit einer bzw. mehreren Acryl- oder Methacrylfunktionen verknüpft sind, verwendet werden.

In einer weiteren vorteilhaften Ausgestaltung der Erfindung ist vorgesehen, dass der UV-Absorber die allgemeine Formel III 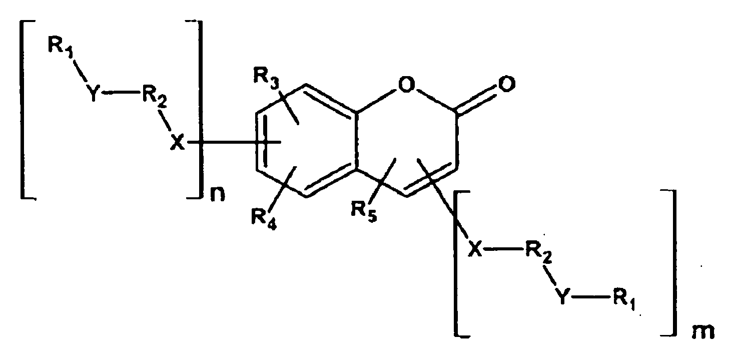 besitzt, wobei
R1: Acryl- bzw. Methacryl-Reste 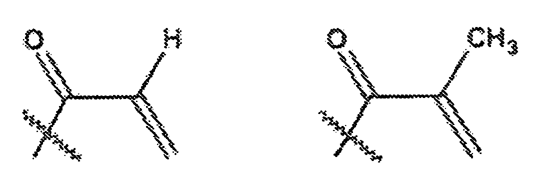 sind,
R2: organische verzweigte und/oder unverzweigte Alkyl- und/oder Aryl-Substituenten mit bis zu 30 Atomen ausgewählt aus C, H, Si, O, N, P, S, F, Cl, Br sind,
R3, R4 und R5: H oder organische verzweigte und/oder unverzweigte Alkyl- und/oder Aryl-Substituenten mit bis zu 30 Atomen ausgewählt aus C, H, Si, O, N, P, S, F, Cl, Br sind,
X, Y: O, S, NH oder NR sind, wobei R ein organischer verzweigter und/oder unverzweigter Alkyl- und/oder Aryl-Substituent mit bis zu 30 Atomen ausgewählt aus C, H, Si, O, N, P, S, F, Cl, Br ist, und
n eine ganze Zahl zwischen 0 und 2 sowie m 0 oder 1 ist, wobei die Summe n+m immer größer gleich 1 ist. Auch hierbei sind alle Stereoisomeren und racemischen Gemische als mitumfasst anzusehen. Beispiele für geeignete Strukturen gemäß Formel III sind:

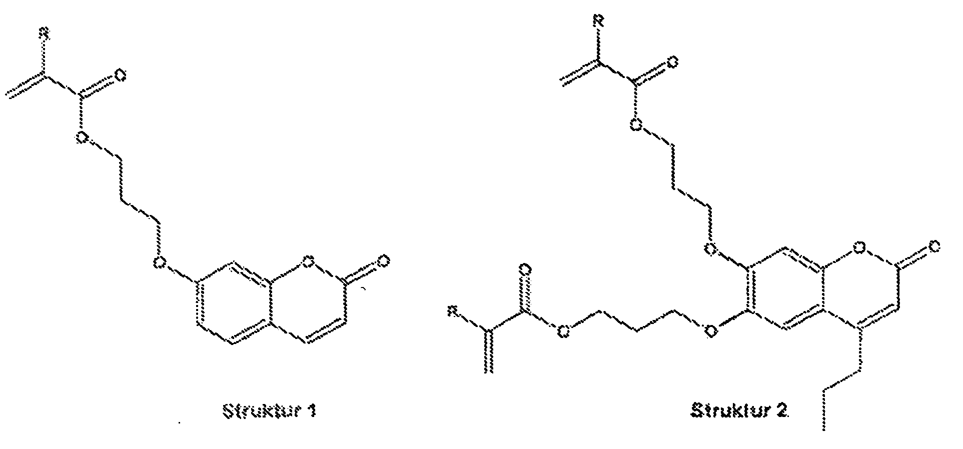

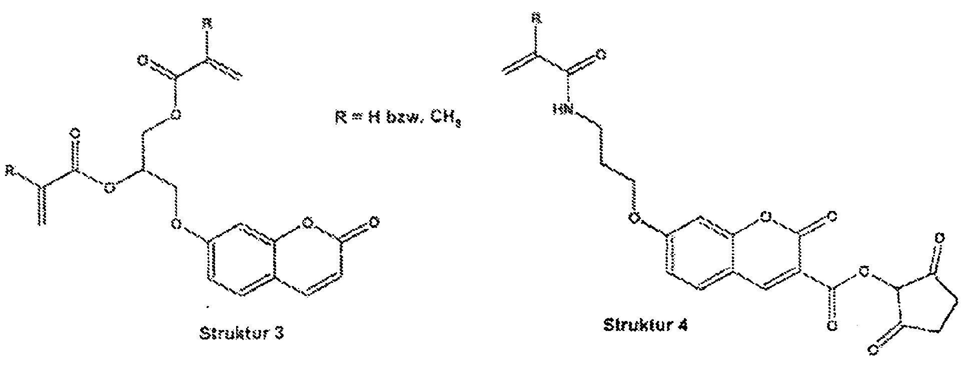

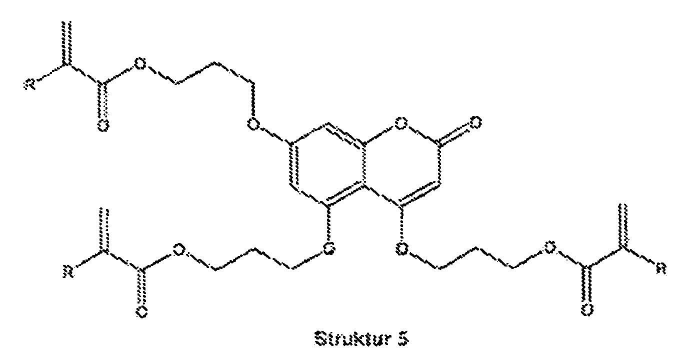

UV-Absorber, deren Grundstruktur auf den Strukturen 2, 3 und 4 basieren, haben den Vorteil, dass diese bedingt durch das Vorhandensein mehrerer polymerisierbarer Endgruppen einen quantitativen Einbau in das Linsenmaterial ermöglichen und darüber hinaus vernetzende Eigenschaften besitzen. Bei einer Linsenherstellung kann so im Idealfall auf die Zugabe eines zusätzlichen Vernetzers verzichtet werden.

Ein bevorzugter UV-Absorber ist Cumarin-7-propoxymethacrylat mit der Struktur:

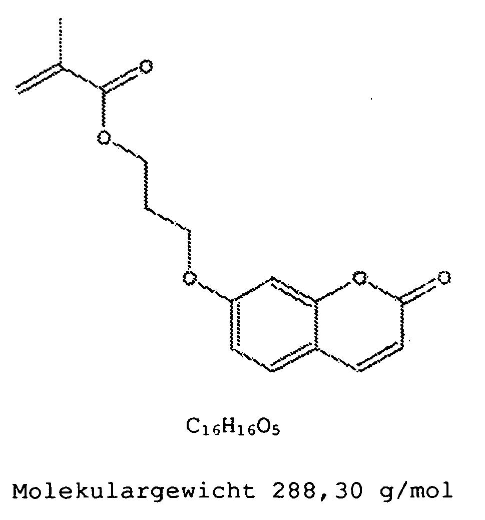

Die Herstellung dieser Verbindung erfolgt in zwei Schritten, wobei das 7-Hydroxycumarin kommerziell erhältlich ist:

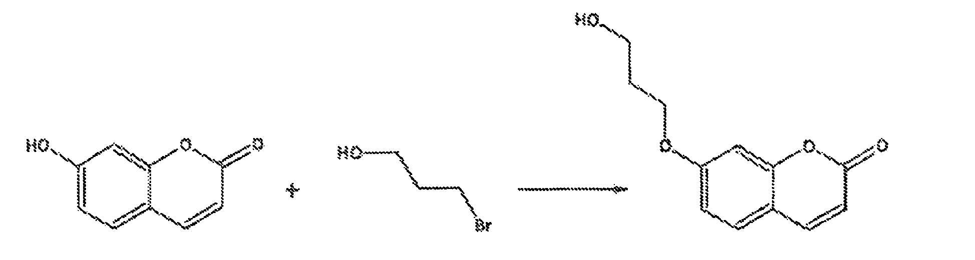

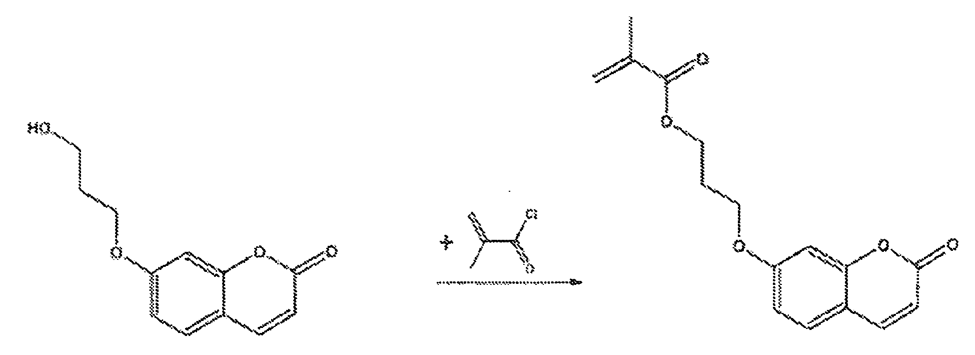

Weitere Ausführungsbeispiele für den UV-Absorber sind Verbindungen bei denen ein Cumarin-Grundkörper über verschiedene Spacer mit einem oder mehreren Acryl- bzw. Methacryl-Resten verbunden ist. Diese besitzen folgende Struktur: 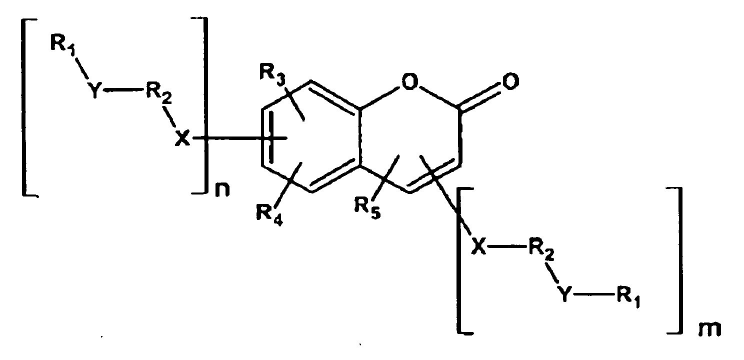 , wobei
R1 : Acryl- bzw. Methacryl-Reste 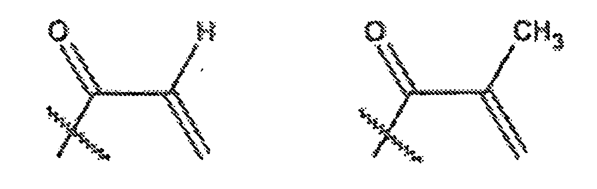 sind,
R2: organische verzweigte und/oder unverzweigte Alkyl- und/oder Aryl-Substituenten mit bis zu 30 Atomen ausgewählt aus C, H, Si, O, N, P, S, F, Cl, Br sind,
R3, R4 und R5: H oder organische verzweigte und/oder unverzweigte Alkyl- und/oder Aryl-Substituenten mit bis zu 30 Atomen ausgewählt aus C, H, Si, O, N, P, S, F, Cl, Br sind,
X, Y: O, S, NH oder NR sind, wobei R ein organischer verzweigter und/oder unverzweigter Alkyl- und/oder Aryl-Substituent mit bis zu 30 Atomen ausgewählt aus C, H, Si, O, N, P, S, F, Cl, Br ist, und
n eine ganze Zahl zwischen 0 und 2 sowie m 0 oder 1 ist, wobei die Summe n+m immer größer gleich 1 ist. Auch hierbei sind alle Stereoisomeren und racemischen Gemische als mitumfasst anzusehen.

Ein Beispiel hierfür ist die Verbindung mit n = 2, m=0, X = O, R2 = C₃H₆, Y = O, R1 = Aryl- bzw. Methacrylrest, R3 = H, R4 = H, R5 = H in der allgemeinen Formel III.

Ein weiteres Ausführungsbeispiel für einen UV-Absorber ist Cumarin-6,7-dipropoxymethacrylat. Auch dieser kann auf einfachem synthetischem Wege analog dem Cumarin-7-propoxymethacrylat in einer 2-stufigen Reaktion dargestellt werden. Das dazu benötigte 6,7-Dihydroxycumarin ist ebenfalls kommerziell verfügbar. Auf diese Weise kann eine Verbindung hergestellt werden, bei der eine zusätzliche Methacrylat-Ankergruppe eingeführt wurde. Das Anbinden dieser zweiten Ankergruppe über einen Alkoxyspacer hat nur einen geringen Einfluss auf die spektralen Eigenschaften des Absorbers, ermöglicht jedoch diesen ebenfalls als Vernetzter bei der Herstellung des Linsenmaterials einzusetzen.

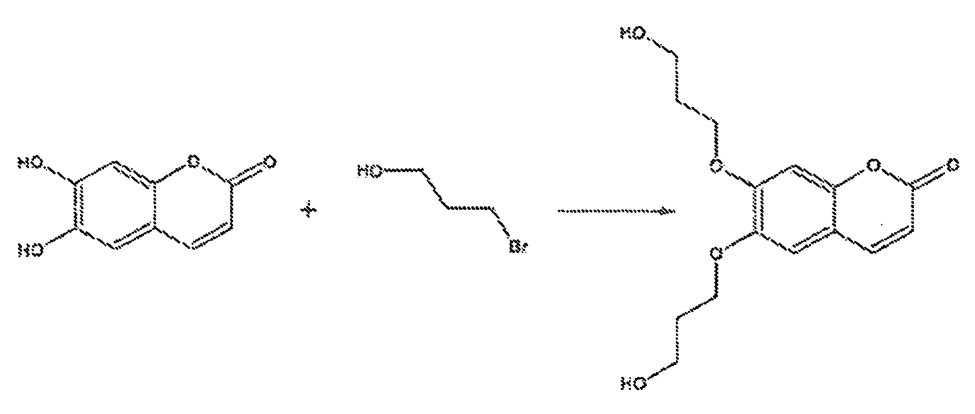

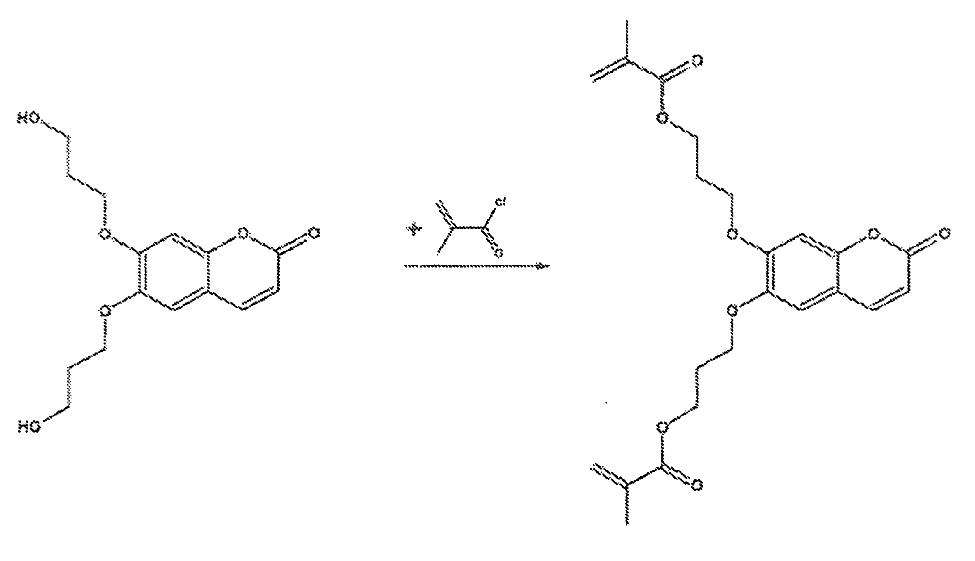

Ein weiteres Beispiel ist eine Struktur mit n = 1, m = 0, X = O, R2 = -CH₂-CH(OR1)CH₂-, Y = O, R1 = Acryl- bzw. Methacrylrest, R3 = H, R4 = H, R5 = H der allgemeinen Formel III.

Eine weitere Möglichkeit einen UV-Absorber mit zwei Ankergruppen herzustellen ergibt sich aus der Verwendung eines verzweigten Dihydroxyhalogenids. Setzt man 7-Hydroxycumarin in einem ersten Schritt mit kommerziell erhältlichem 3-Brom-1,2-propandiol um und acryliert bzw. methacryliert das entstandene Alkoxydiol nachfolgend, erhält man einen weiteren bifunktionalen UV-Absorber.

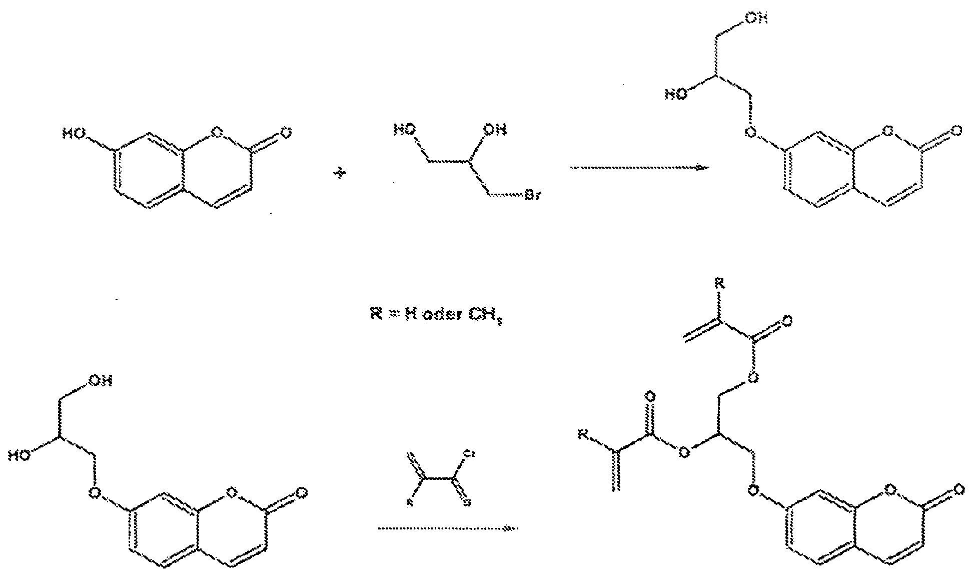

Ein weiteres Beispiel ist eine Struktur mit n = 1, m = 0, X = O, R² = -CH₂-CH(OR1)CH₂-, Y = O, R1 = Methacrylrest, R3 = H, R4 = H, R5 = H der allgemeinen Formel III.

Setzt man 7-Hydroxycumarin nicht mit Acrylsäure bzw. Methacrylsäurechlorid, sondern mit kommerziell erhältlichem Methacrylsäureglycidylester um, so erhält man in einem einzigen Reaktionsschritt einen weiteren UV-Filter, in dem der Cumarin-Grundkörper durch eine aliphatische Kette vom Methacrylatrest getrennt ist. Durch nachfolgende Veresterung mit Methacryloylchlorid kann eine weitere Methacrylatfunktion an der sekundären Alkoholgruppe eingeführt werden.

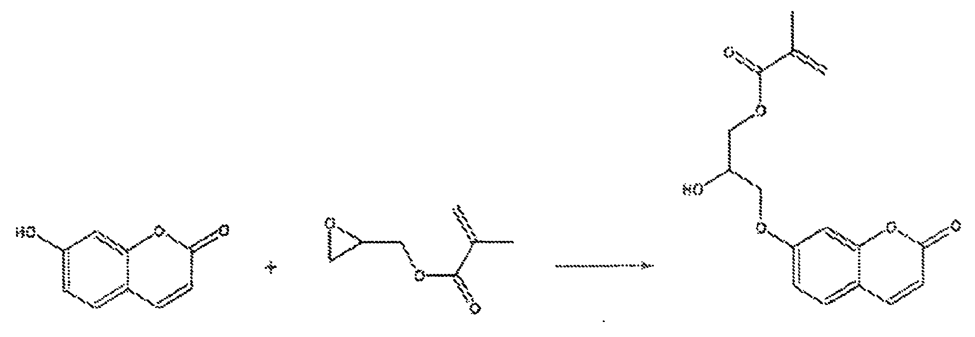

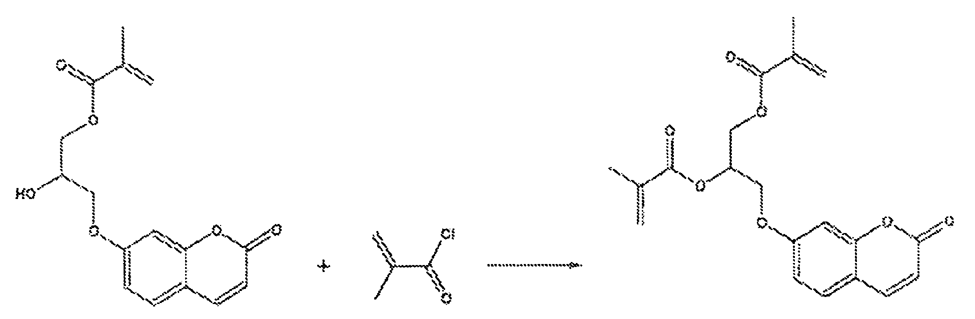

Ein weiteres Beispiel ist eine Struktur mit n = 1, m = 0, X = O, R2 = C₃H₆, Y = O, R1 = Acryl- bzw. Methacrylrest, R3 = H, R4 = H, R5 = C₃H₇ der allgemeinen Formel III. Hier ist R5 eine Propylgruppe, die einen schwachen induktiven Effekt (+I-Effekt) besitzt. Das Einbringen einer zusätzlichen Propylgruppe in den zuvor beschriebenen bevorzugten UV-Absorber ist synthetisch ohne Probleme zu bewältigen und verändert die spektralen Eigenschaften des Chromophors nur in geringem Maße. Setzt man bei der Synthese nicht 7-Hydroxycumarin sondern das ebenfalls kommerziell erhältliche 7-Hydroxy-4-propylcoumarin ein, erhält man nach der Methacrylierung ein Cumarinderivat, dass sich vom bevorzugten UV-Absorber um nur eine Propylseitenkette unterscheidet.

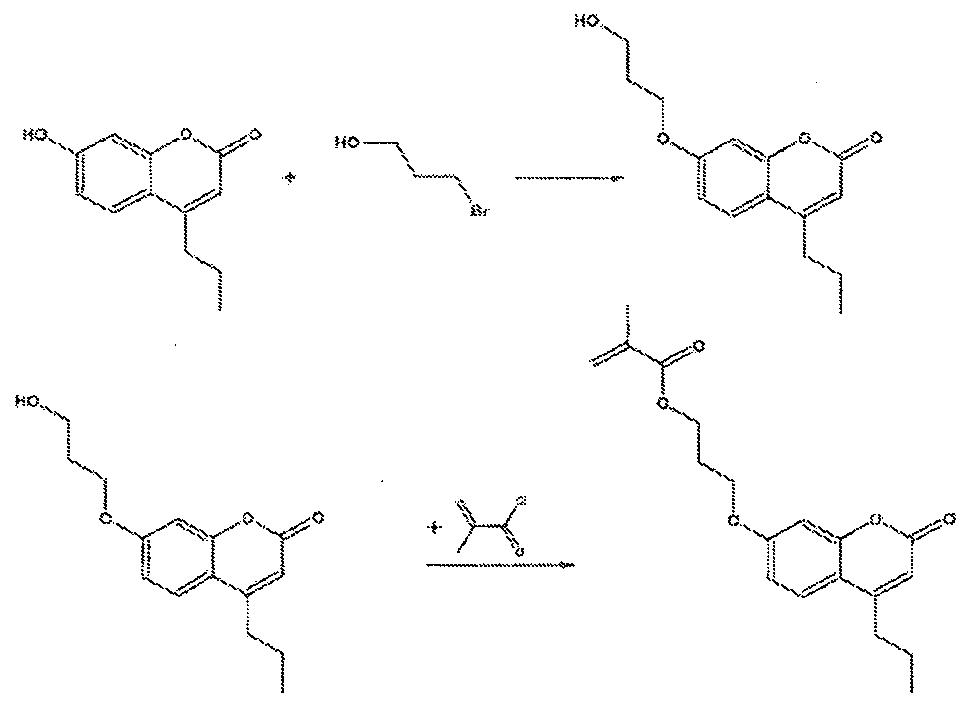

Ein weiteres Beispiel ist eine Struktur mit n = 2, m = 1, X = O, R2 = C₃H₆, Y = O, R1 = Acryl- bzw. Methacrylrest, R3 = H, R4 = H, R5 = H der allgemeinen Formel III.

Auch ein trifunktionaler UV-Absorber lässt sich auf einfachem synthetischem Wege herstellen. Ausgehend vom 4,5,7-Trihydroxycumarin erhält man nach der Alkoxylierung mit 3-Brom-1-propanol und nachfolgender Acrylierung bzw. Methacrylierung einen UV-Absorber mit drei Ankergruppen. 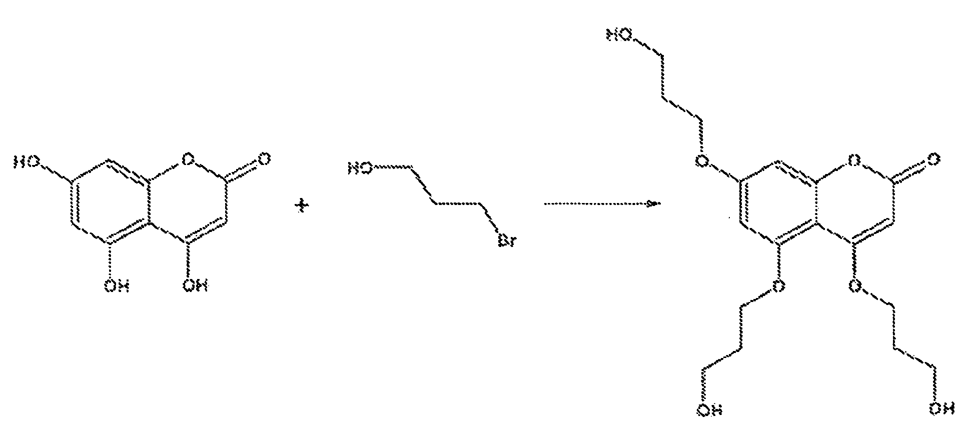 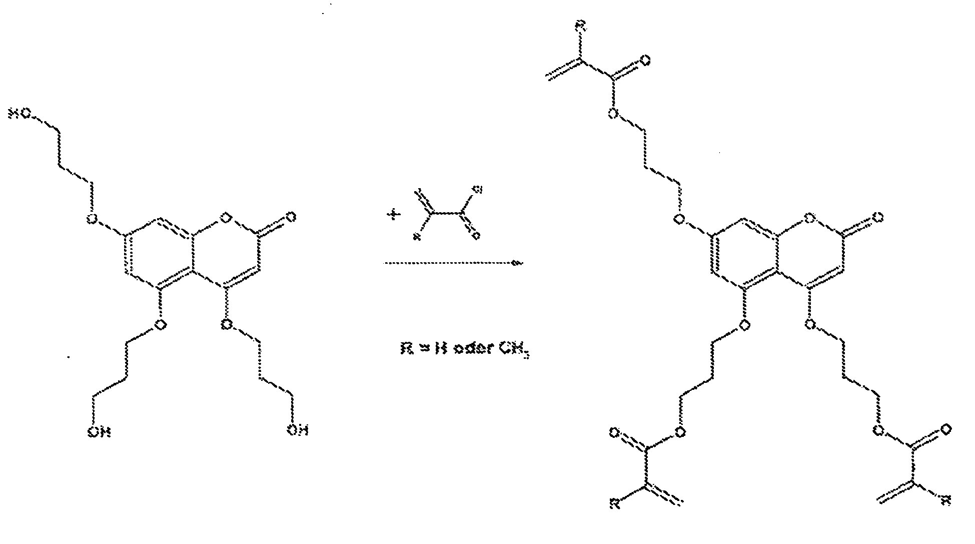 Es ist weiter bevorzugt, dass das Copolymer einen Violett-Absorber (Gelbfarbstoff) enthält. Das Copolymer enthält vorzugsweise einen Violett-Absorber, der violettes Licht der Wellenlängen von etwa 400 nm bis 430 nm absorbiert und vorzugsweise im wesentlichen quantitativ oder besonders bevorzugt quantitativ absorbiert.

In einer weiteren vorteilhaften Ausgestaltung der Erfindung ist vorgesehen, dass der Violett-Absorber die allgemeine Formel IV 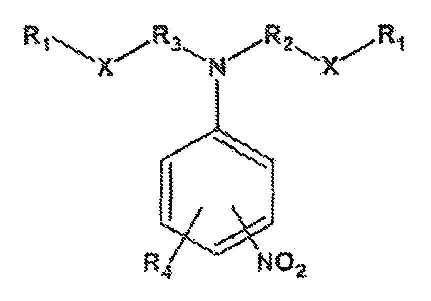 besitzt, wobei
R1: Acryl- bzw. Methacryl-Reste sind,
R2: eine organische verzweigte und/oder unverzweigte Alkyl- und/oder Aryl-Spacergruppe mit bis zu 30 Atomen ausgewählt aus C, H, Si, O, N, P, S, F, Cl, Br ist,
R3: eine organische verzweigte und/oder unverzweigte Alkyl- und/oder Aryl-Spacergruppe mit bis zu 30 Atomen ausgewählt aus C, H, Si, O, N, P, S, F, Cl, Br ist,
R4: H oder ein organischer verzweigter und/oder unverzweigter Alkyl- und/oder Aryl-Substituent mit bis zu 30 Atomen ausgewählt aus C, H, Si, O, N, P, S, F, Cl, Br ist, und
X: O, S, NH oder NR ist, wobei R ein organischer verzweigter und/oder unverzweigter Alkyl- und/oder Aryl-Substituent mit bis zu 30 Atomen ausgewählt aus C, H, Si, O, N, P, S, F, Cl, Br ist.

Auch hierbei sind alle Stereoisomeren und racemischen Gemische als mitumfasst anzusehen. Beispiele entsprechender Strukturen sind:

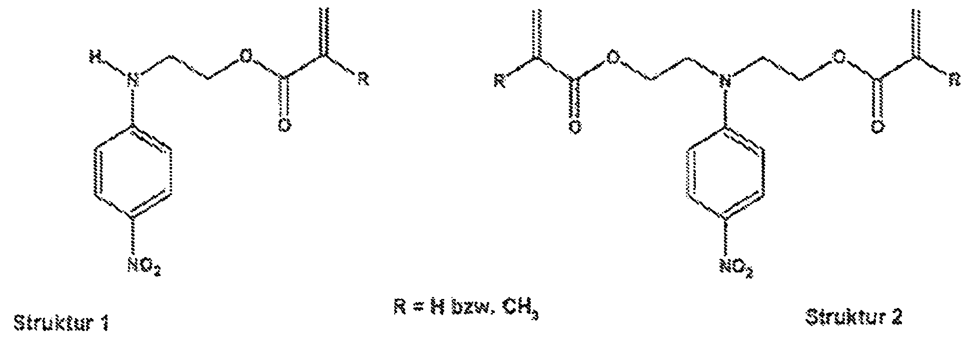

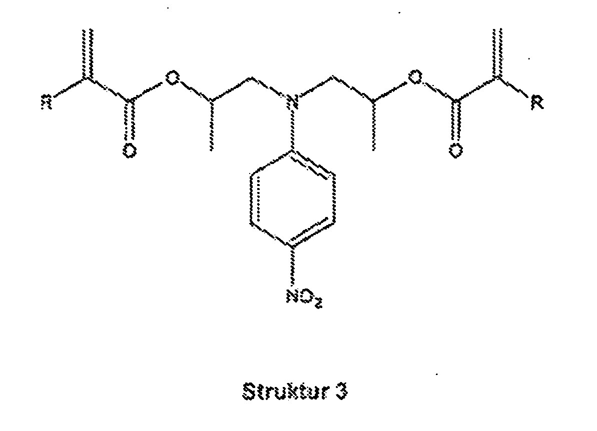

In einer weiteren vorteilhaften Ausgestaltung der Erfindung ist vorgesehen, dass der Violett-Absorber die allgemeine Formel V 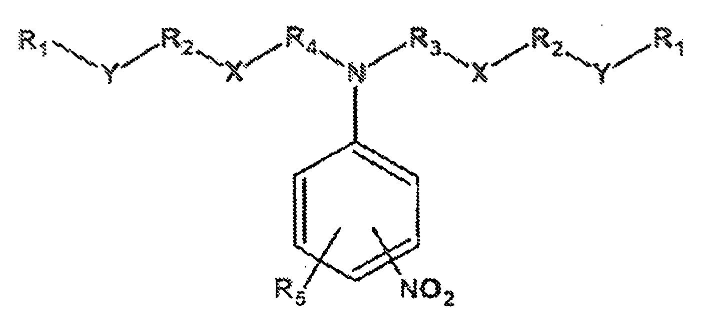 besitzt, wobei
R1: Acryl- bzw. Methacryl-Reste sind,
R2: organische verzweigte und/oder unverzweigte Alkyl- und/oder Aryl-Spacergruppen mit bis zu 30 Atomen ausgewählt aus C, H, Si, O, N, P, S, F, Cl, Br sind,
R3: eine organische verzweigte und/oder unverzweigte Alkyl- und/oder Aryl-Spacergruppe mit bis zu 30 Atomen ausgewählt aus C, H, Si, O, N, P, S, F, Cl, Br ist,
R4: ein organischer verzweigter und/oder unverzweigter Alkyl- und/oder Aryl-Substituent mit bis zu 30 Atomen ausgewählt aus C, H, Si, O, N, P, S, F, Cl, Br ist,
R5: H oder ein organischer verzweigter und/oder unverzweigter Alkyl- und/oder Aryl-Substituent mit bis zu 30 Atomen ausgewählt aus C, H, Si, O, N, P, S, F, Cl, Br ist,
X, Y: O, S, NH oder NR sind, wobei R ein organischer verzweigter und/oder unverzweigter Alkyl- und/oder Aryl-Substituent mit bis zu 30 Atomen ausgewählt aus C, H, Si, O, N, P, S, F, Cl, Br ist.

Auch hierbei sind alle Stereoisomeren und racemischen Gemische als mitumfasst anzusehen. Beispiele entsprechender Strukturen (alle Stereoisomere oder racemische Gemische sind beinhaltet) sind:

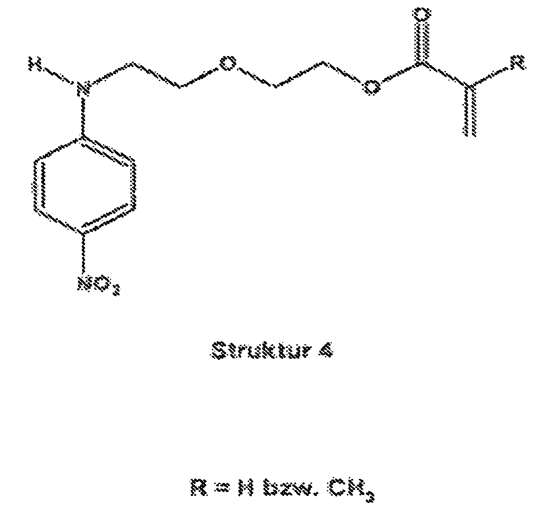

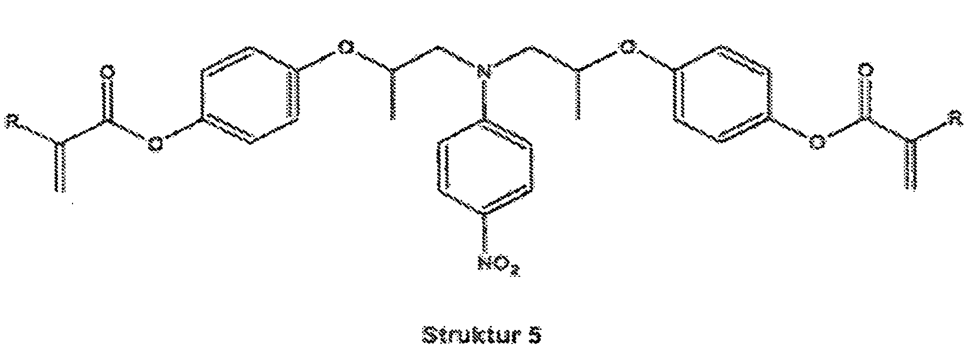

Ein bevorzugter Farbstoff für den Violett-Absorber ist N, N-Di-2'-ethylmethacrylat-4-nitroanilin
mit der Struktur:

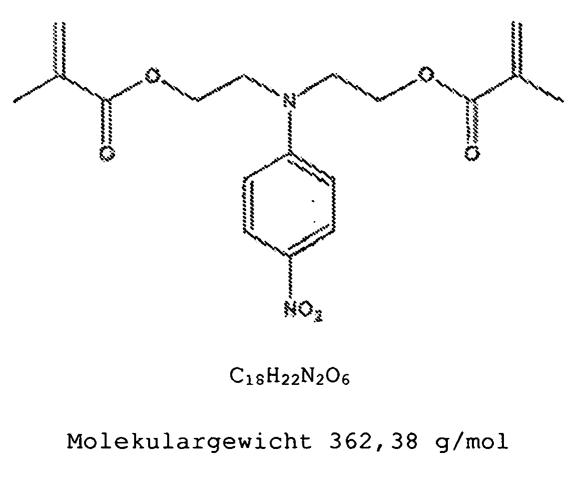

Die Herstellung dieser Verbindung erfolgt in zwei Schritten (gemäß Offenlegungsschrift EP 0321891 A2), wobei beide Edukte, sowohl das 4-Fluornitrobenzol, als auch das Diethanolamin kommerziell erhältlich sind:

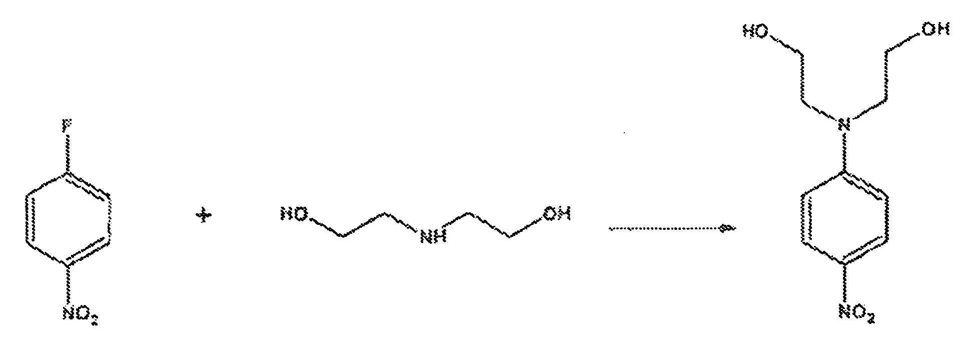

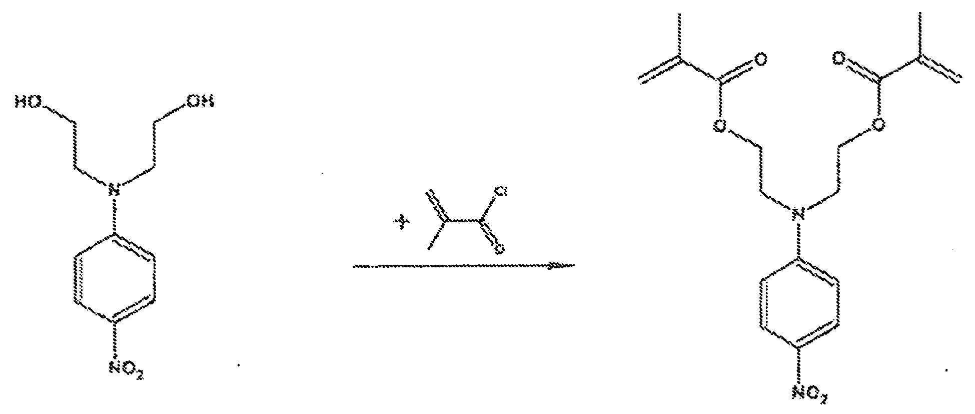

Die Methacryl- Reste dienen zur kovalenten Einbindung des Violett-Filters in das Copolymer bzw. ein Trägermaterial, insbesondere Linsenmaterial auf Acrylat-Basis. Aufgrund der Bifunktionalität verläuft der Einbau quantitativ und somit deutlich effektiver als bei den am Markt erhältlichen monofunktionalen Violett-Filtern.

Weitere Ausführungsbeispiele für den Violett-Absorber sind ebenfalls Verbindungen, bei denen ein Nitroanilin Grundkörper über verschiedene Spacer mit einem oder mehreren Acryl- bzw. Methacryl-Resten verbunden ist.

Ein weiteres Beispiel ist eine Struktur mit R2 und R3 = - CH₂-CH(CH₃)-, X = O, R1 = Acryl- oder Methacrylrest, R4 = H der allgemeinen Formel IV.

Ein weiteres Ausführungsbeispiel für einen gelben Chromophor/Violett-Filter ist das N,N-Di-2'-isopropylmethacrylat-4-nitroanilin. Auch dieses kann auf einfachem synthetischem Wege analog dem Diethylmethacrylat-4-nitroanilin in einer 2-stufigen Reaktion hergestellt werden. Das dazu benötigte Diisopropanolamin ist ebenfalls kommerziell verfügbar. Auf diese Weise kann eine Verbindung hergestellt werden, die sich vom bevorzugten Filter um nur jeweils eine CH₃-Gruppe in der Seitenkette unterscheidet. Durch den positiven induktiven Effekt der Methylgruppen absorbiert dieser Chromophor geringfügig zu längeren Wellenlängen verschoben.

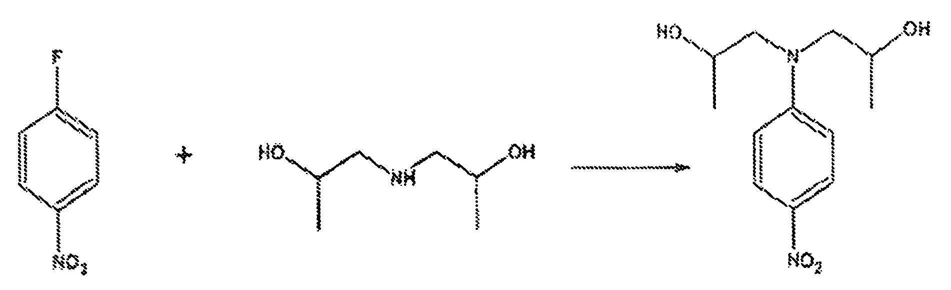

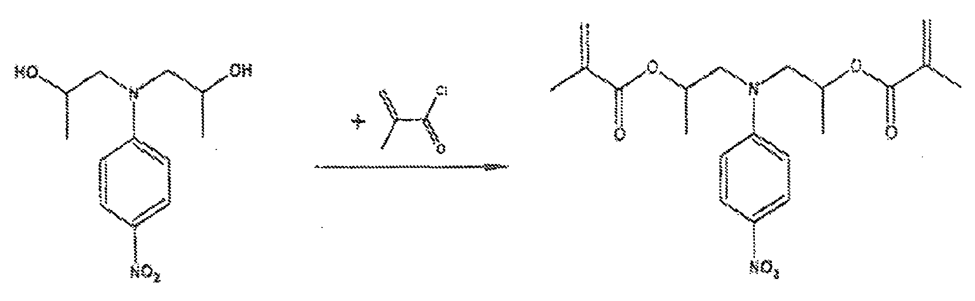

Ein weiteres Beispiel ist eine Struktur mit R2 und R3 = C₂H₄, X = NR, R1 = Acryl- oder Methacrylrest, R4 = H der allgemeinen Formel IV.

Bei diesem Beispiel wird N,N-Dihydroxyethyl-4-nitroanilin durch eine einfache synthetische Methode in das Diamino-Derivat umgesetzt. Dieses Diamin kann durch eine Reaktion mit Acrylsäurechlorid in das Diamid umgewandelt werden. Die Struktur des Chromophors bleibt unverändert und ist durch zwei Methyleneinheiten vom Acryl-Amid getrennt.

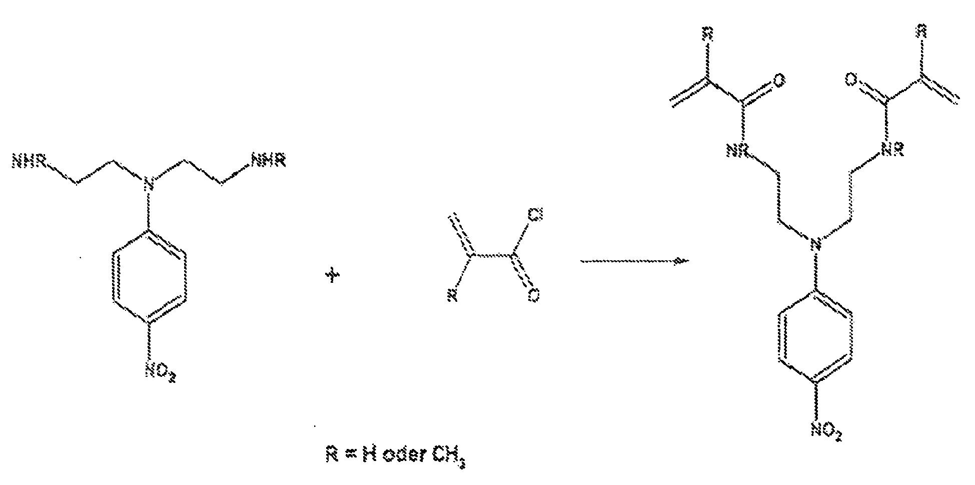

Ein weiteres Beispiel ist eine Struktur mit R3 und R4 = C₂H₄, X = O, R2 = -CH₂-CH(OH)CH₂-, Y = O, R1 = Acryl- oder Methacrylrest, R5 = H der allgemeinen Formel V.

Setzt man N,N-Dihydroxyethyl-4-nitroanilin nicht mit Acrylsäure bzw. Methacrylsäurechlorid, sondern mit kommerziell erhältlichem Methacrylsäureglycidylester um, so erhält man in einem einzigen Reaktionsschritt einen weiteren Violett-Filter, in dem der Chromophor durch aliphatische Ketten von den Methacrylatresten getrennt ist.

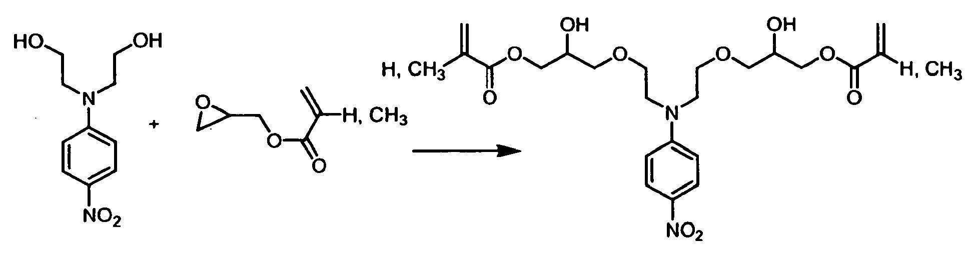

Ein weiteres Beispiel ist eine Struktur mit R2 und R3 = C₂H₄, X = O, R1 = Acryl- oder Methacrylrest, R4 = CH₃ der allgemeinen Formel IV.

Hier ist R4 eine Methylgruppe, die einen schwachen induktiven Effekt (+I-Effekt) besitzt. Das Einbringen einer zusätzlichen Methylgruppe in den zuvor beschriebenen bevorzugten Violett-Filter ist synthetisch ohne Probleme zu bewältigen und verändert die spektralen Eigenschaften des Chromophors nur in geringem Maße. Setzt man Diethanolamin nicht mit 4-Fluornitrobenzen, sondern mit dem ebenfalls kommerziell erhältlichen 2-Fluor-5-nitrotoluol um, so wird ein Nitroanilin erzeugt, das sich vom bevorzugten Violett-Absorber um gerade eine zusätzliche Methylgruppe am Anilin-Ring unterscheidet. Durch Veresterung mit Acryloylchlorid oder Methacryloylchlorid wird so ein weiterer Chromophor mit den gewünschten spektralen Eigenschaften erhalten.

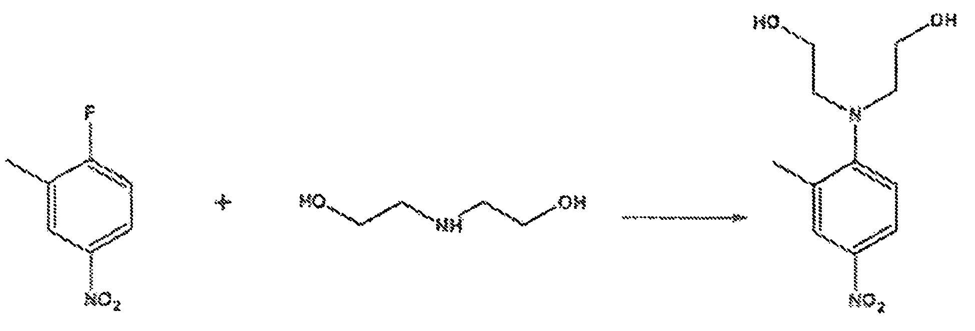

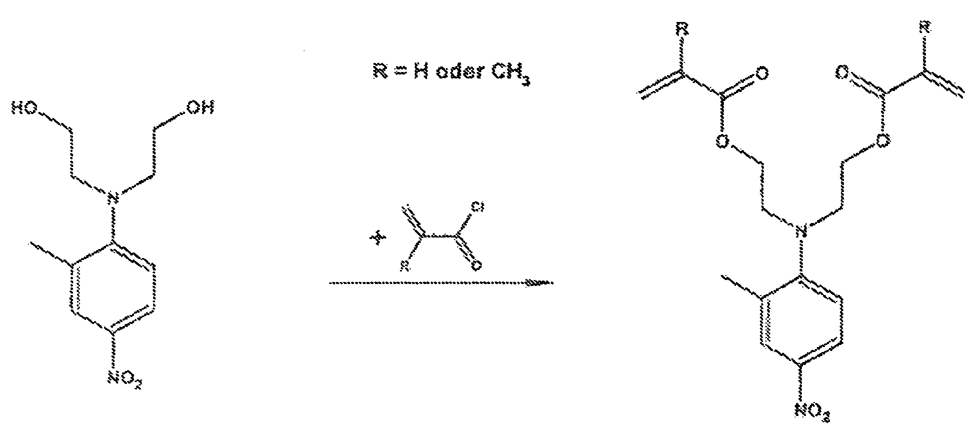

Als biokompatibles Trägermaterial sind Acrylate, insbesondere mit einem Wassergehalt von 1% bis 30%, für die ophthalmologische Zusammensetzung geeignet. In dem Copolymer bzw. in diesem Trägermaterial sind der UV-Absorber bzw. der Violett-Absorber kovalent eingebunden. Der UV-Absorber ist vorzugsweise in einem Konzentrationsbereich von 0,5 % bis 1,0 % enthalten. Wenn die ophthalmologische Zusammensetzung für eine IOL verwendet wird, hängt die jeweilige Konzentration des UV-Absorbers vom jeweiligen Scheitelbrechwert (Dioptrie) der Linse ab. Der Violett-Absorber ist ebenfalls kovalent im Acrylat-Trägermaterial bzw. im Copolymer gebunden. Er kann in einem Konzentrationsbereich von 0,03% bis 0,16% vorliegen. Auch hier hängt bei der Verwendung der ophthalmologischen Zusammensetzung für eine IOL die Konzentration des Violett-Absorbers direkt von der Dioptrie der Linse ab.

Die Gefahr des Auswaschens der Absorber aus der Trägermatrix besteht nicht, da sowohl der erfindungsgemäße UV-Absorber als auch der Violett-Filter bedingt dadurch, dass sie zwei polymerisationsfähige Endgruppen tragen, quantitativ in das Linsenmaterial einbauen.

Geeignete biokompatible Trägermaterialien für den UV-Absorber bzw. den Violett-Absorber sind beispielsweise Hydroxyethylmethacrylat (HEMA), Methylmethacrylat (MMA), Ethoxyethylmethacrylat (EOEMA), Ethoxyethoxyethylacrylat (EEEA), Tetrahydrofufurylmethacrylat (THFMA), Tetrahydrofufurylacrylat (THFA), 2-Hydroxypropylmethacrylat (HPMA), 2-Hydroxypropylacrylat (HPA), 2-Hydroxyethylacrylamid, 2-Hydroxyethylmethacrylamid, Methoxyethylmethacrylat (MOEMA) und Methoxyethylacrylat (MOEA). Aus den zuvor genannten Stoffen können Copolymerisate, evtl. unter Verwendung eines Quervemetzers, hergestellt und als Trägermaterial verwendet werden. Die prozentuale Zusammensetzung der Monomere ist in einem weiten Bereich variabel. Die Trägermaterialien können hydrophil mit einem Wassergehalt von beispielsweise 1% bis 30% oder hydrophob eingestellt werden. Begrenzender Faktor bei hydrophoben, wasserfreien Polymeren ist der Glaspunkt. Dieser kann im Bereich zwischen 0°C und 11°C liegen. Darüber hinaus ist wichtig, dass hydrophile Polymere nach der Quellung eine ausreichende Flexibilität aufweisen.

Erfindungsgemäß besonders bevorzugt ist, dass im erfindungsgemäßen Copolymer wenigstens ein Teil des Vernetzers oder der Vernetzer ein Violett-Absorber oder ein UV-Absorber ist.

Dies hat den Vorteil, dass nicht nur die Menge der eingesetzten verschiedenen Chemikalien verringert werden kann, sondern auch, dass auf diese Weise der entsprechende Absorber durch kovalente Bindung Medialbestandteil des Copolymers ist, was eine Freisetzung des entsprechenden Absorbers in vielen Anwendungen minimiert.

Das Copolymer weist vorzugsweise einen Brechungsindex von wenigstens 1,3 auf.

Die ophthalmische Linse ist vorzugsweise eine Intraokularlinse und/oder ein ophthalmisches Implantat. Weiter bevorzugt kann die ophthalmische Linse einteilig oder mehrteilig sein. In einer weiter bevorzugten Ausführungsform ist die ophthalmische Linse faltbar.

Weiterhin kann eine ophtalmologische Zusammensetzung vorgesehen sein, welche einen UV-Absorber aufweist, der Strahlung im Wellenlängenbereich von etwa 200 nm bis 400 nm quantitativ absorbiert. Ferner beinhaltet die ophthalmologische Zusammensetzung einen Violett-Absorber, der violettes Licht der Wellenlängen von etwa 400 nm bis 430 nm absorbiert. Geeignete Chromophorgrundstrukturen des Violett-Absorbers sind N-alkoxyacrylierte bzw. N-alkoxymethacrylierte oder aber auch N,N-dialkoxyacrylierte bzw. N,N-dialkoxymethacrylierte Nitroaniline.

Als UV-Absorber beinhaltet die ophthalmologische Zusammensetzung ein biokompatibles UV-Lichtschutzmittel, wofür Cumarinderivate, die gegebenenfalls über Alkylspacer mit einer bzw. mehreren Acryl- oder Methacrylfunktionen verknüpft sind, verwendet werden.

Vorzugsweise ist die Zusammensetzung ausschließlich auf Acrylat- und/oder Methacrylatbasis aufgebaut.

Geeignete UV-Absorber der ophthalmologischen Zusammensetzung sind Verbindungen der folgenden Strukturen: 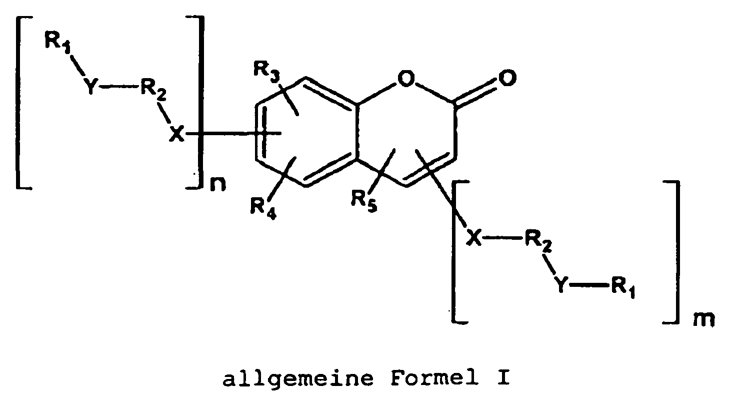 n =0 bis 2
m = 0 oder 1, wobei n+m≥ 1
X= O, NH, NR6
Y= O, NH, NR6
R1: Acryl- oder Methacryl-Rest 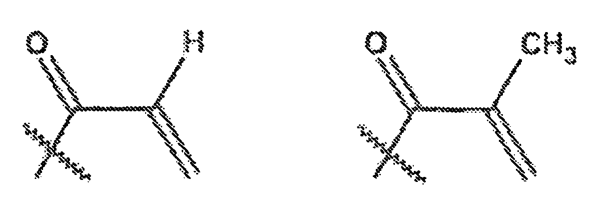 R2: organische Alkyl- und/oder Aryl-Spacergruppe mit bis zu 30 Atomen, ausgewählt aus C, H, Si, O, N, P, S, F, Cl, Br,
R3, R5, R6: H oder organische Alkyl- oder Aryl-gruppe (oder Kombination aus beiden) mit bis zu 30 Atomen ausgewählt aus C, H, Si, O, N, P, S, F, Cl, Br
R4 = nur wenn n =0 oder 1: H oder organische Alkyl- oder Arylgruppe (oder Kombinationen aus beiden) mit bis zu 30 Atomen, ausgewählt aus: C, H, Si, O, N, P, S, Cl, Br, F.

Beispiele entsprechender Strukturen (alle Stereoisomere oder racemische Gemische sind beinhaltet) sind:

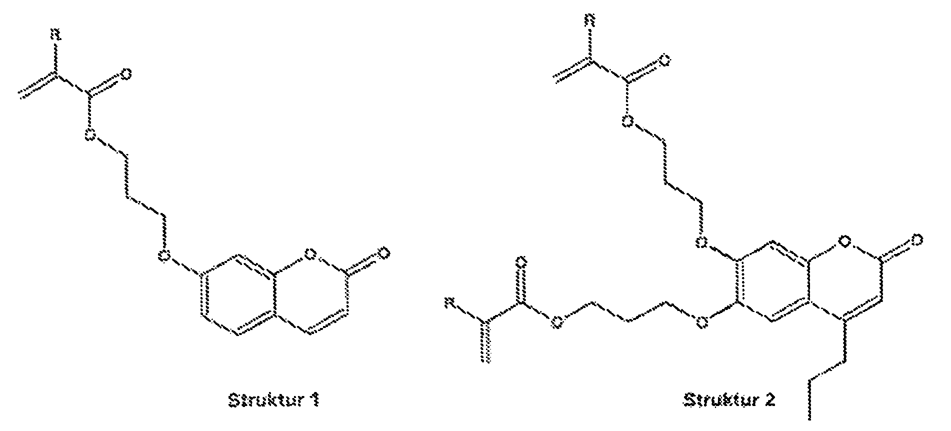

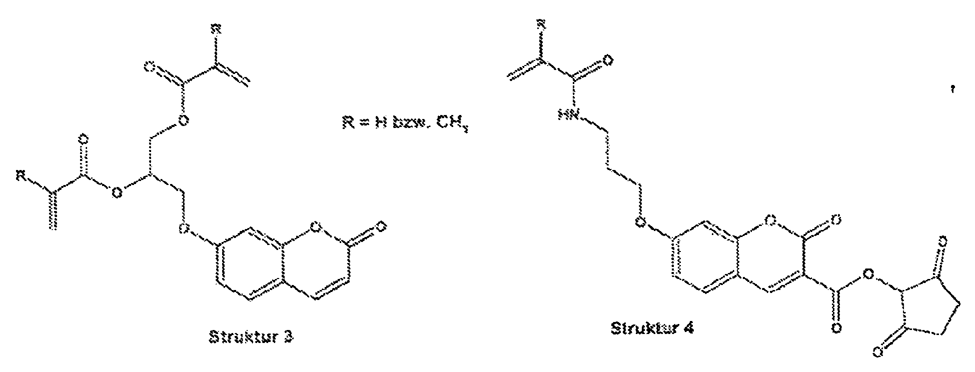

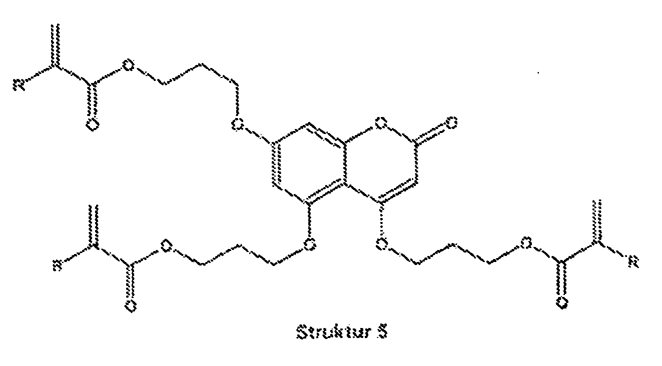

UV-Absorber, deren Grundstruktur auf den Strukturen 2, 3 und 4 basieren, haben den Vorteil, dass diese bedingt durch das Vorhandensein mehrerer polymerisierbarer Endgruppen einen quantitativen Einbau in das Linsenmaterial ermöglichen und darüber hinaus vernetzende Eigenschaften besitzen. Bei einer Linsenherstellung kann so im Idealfall auf die Zugabe eines zusätzlichen Vernetzers verzichtet werden.

Ein bevorzugter UV-Absorber ist Cumarin-7-propoxymethacrylat mit der Struktur:

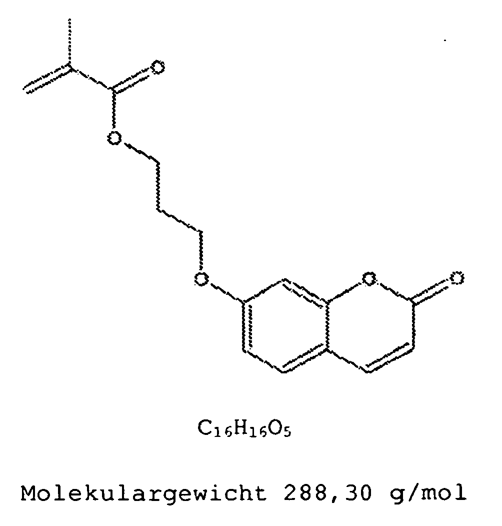

Die Herstellung dieser Verbindung erfolgt in zwei Schritten, wobei das 7-Hydroxycumarin kommerziell erhältlich ist:

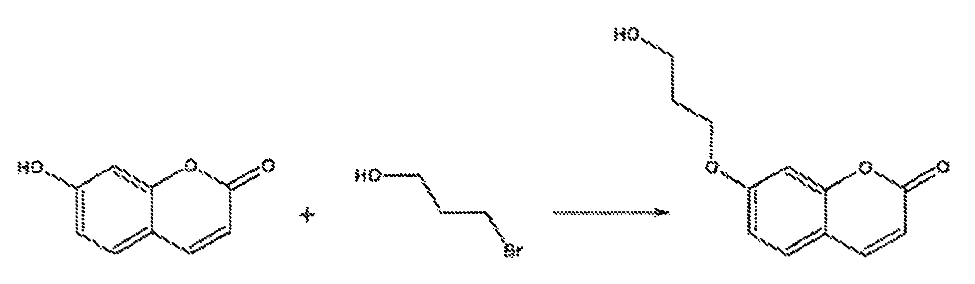

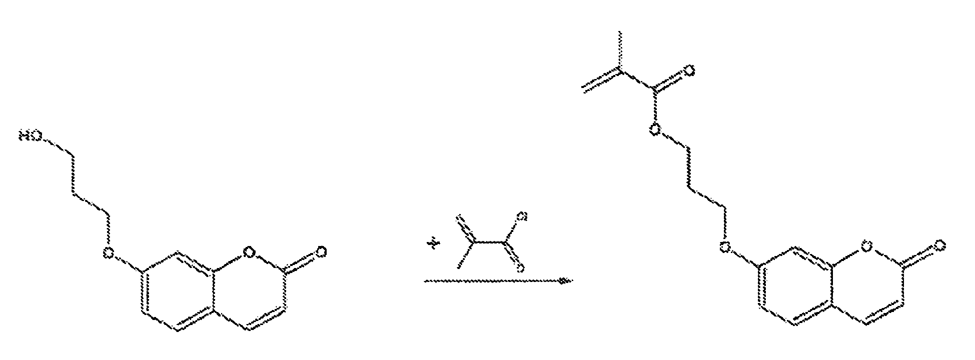

Weitere Ausführungsbeispiele für den UV-Absorber sind Verbindungen bei denen ein Cumarin-Grundkörper über verschiedene Spacer mit einem oder mehreren Acryl- bzw. Methacryl-Resten verbunden ist. Diese besitzen folgende Struktur: 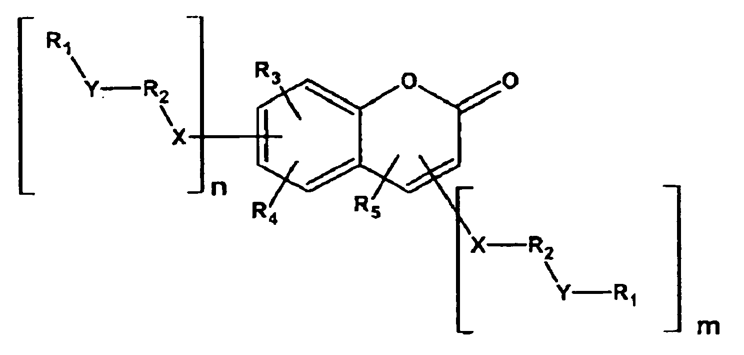 , wobei
R1: ein Acryl- oder Methacryl-Rest ist
R2: organische verzweigte und unverzweigte Alkyl- und/oder Aryl-Substituenten (oder Kombinationen von beiden) mit bis zu 30 Atomen ausgewählt aus C, H, Si, O, N, P, S, F, Cl, Br
R3, R4 und R5: H oder organische verzweigte und unverzweigte Alkyl- und/oder Aryl-Substituenten (oder Kombinationen von beiden) mit bis zu 30 Atomen ausgewählt aus C, H, Si, O, N, P, S, F, Cl, Br
X und Y: O, S, NH, NR (R ist ein organischer verzweigter oder unverzweigter Alkyl- und/oder Aryl-Substituent (oder Kombinationen von beiden) mit bis zu 30 Atomen ausgewählt aus C, H, Si, O, N, P, S, F, Cl, Br)
n = 0 bis 2 sowie m = 0 oder 1 wobei n+m immer größer gleich 1 ist.

### Ausführungsbeispiele UV-Absorber

### Beispiel 1:

n = 2, m = 0, X - O, R2 = C₃H₆, Y = O, R1 = Acryl- bzw. Methacrylrest, R3 = H, R4 = H, R5 = H in der allgemeinen Formel I.

Ein weiteres Ausführungsbeispiel für einen UV-Absorber im Sinne der ophthalmologischen Zusammensetzung ist Cumarin-6,7-dipropoxymethacrylat. Auch dieser kann auf einfachem synthetischem Wege analog dem Cumarin-7-propoxymethacrylat in einer 2-stufigen Reaktion dargestellt werden. Das dazu benötigte 6,7-Dihydroxycumarin ist ebenfalls kommerziell verfügbar. Auf diese Weise kann eine Verbindung hergestellt werden, bei der eine zusätzliche Methacrylat-Ankergruppe eingeführt wurde. Das Anbinden dieser zweiten Ankergruppe über einen Alkoxyspacer hat nur einen geringen Einfluss auf die spektralen Eigenschaften des Absorbers, ermöglicht jedoch diesen ebenfalls als Vernetzter bei der Herstellung des Linsenmaterials einzusetzen.

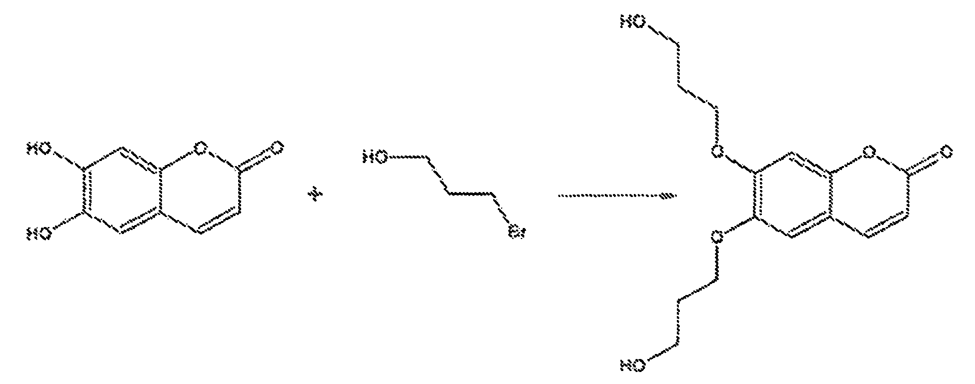

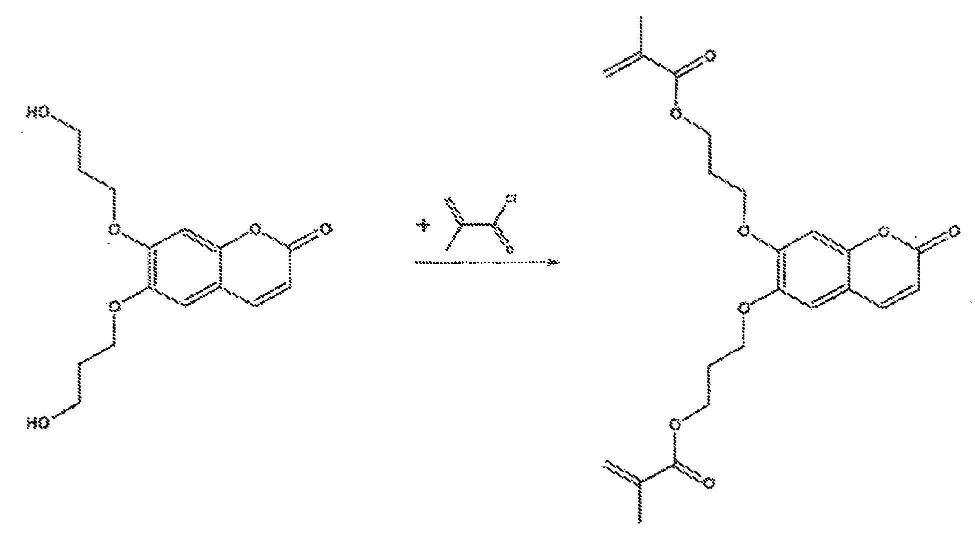

### Beispiel 2:

n = 1, m = 0, X = O, R2 = -CH₂-CH(OR1)CH₂-, Y = O, R1 = Acryl- bzw. Methacrylrest, R3 = H, R4 = H, R5 = H der allgemeinen Formel I.

Eine weitere Möglichkeit einen UV-Absorber mit zwei Ankergruppen herzustellen ergibt sich aus der Verwendung eines verzweigten Dihydroxyhalogenids. Setzt man 7-Hydroxycumarin in einem ersten Schritt mit kommerziell erhältlichem 3-Brom-1,2-propandiol um und acryliert bzw. methacryliert das entstandene Alkoxydiol nachfolgend, erhält man einen weiteren bifunktionalen UV-Absorber.

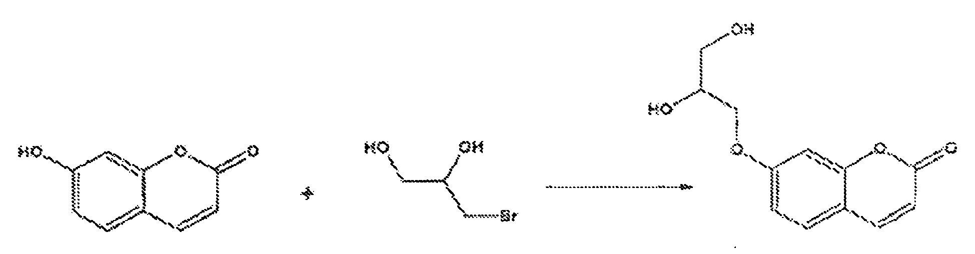

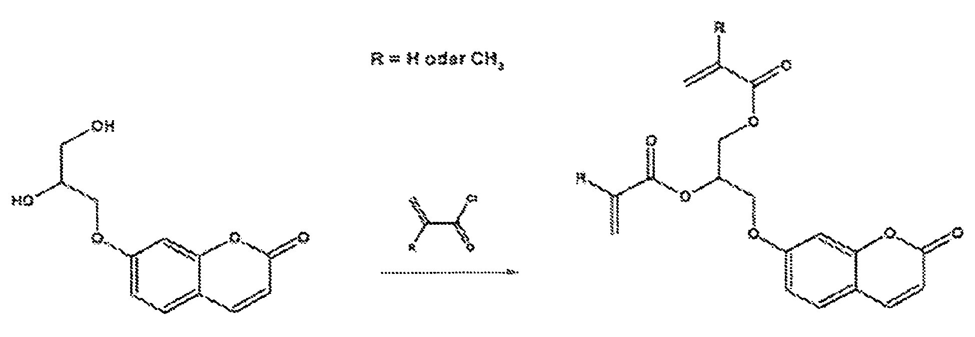

### Beispiel 3:

n = 1, m = 0, X = 0, R2 = -CH₂-CH(OR1)CH₂-, Y = O, R1 = Methacrylrest, R3 = H, R4 = H, R5 = H der allgemeinen Formel I.

Setzt man 7-Hydroxycumarin nicht mit Acrylsäure bzw. Methacrylsäurechlorid, sondern mit kommerziell erhältlichem Methacrylsäureglycidylester um, so erhält man in einem einzigen Reaktionsschritt einen weiteren UV-Filter, in dem der Cumarin-Grundkörper durch eine aliphatische Kette vom Methacrylatrest getrennt ist. Durch nachfolgende Veresterung mit Methacryloylchlorid kann eine weitere Methacrylatfunktion an der sekundären Alkoholgruppe eingeführt werden.

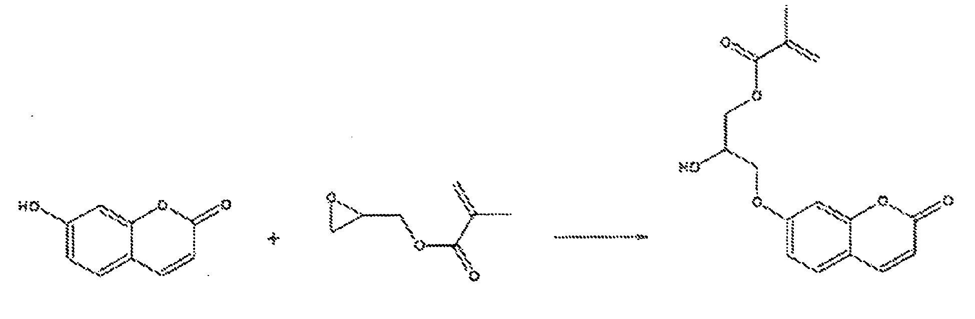

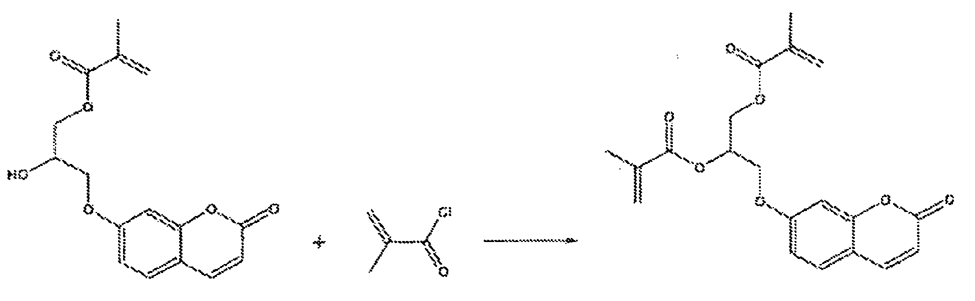

### Beispiel 4:

n = 1, m = 0, X = 0, R2 = C₃H₆, Y = O, R1 = Acryl- bzw. Methacrylrest, R3 = H, R4 = H, R5 = C₃H₇ der allgemeinen Formel I.

Hier ist R5 eine Propylgruppe, die einen schwachen induktiven Effekt (+I-Effekt) besitzt. Das Einbringen einer zusätzlichen Propylgruppe in den zuvor beschriebenen bevorzugten UV-Absorber ist synthetisch ohne Probleme zu bewältigen und verändert die spektralen Eigenschaften des Chromophors nur in geringem Maße. Setzt man bei der Synthese nicht 7-Hydroxycumarin sondern das ebenfalls kommerziell erhältliche 7-Hydroxy-4-propylcoumarin ein, erhält man nach der Methacrylierung ein Cumarinderivat, dass sich vom bevorzugten UV-Absorber um nur eine Propylseitenkette unterscheidet.

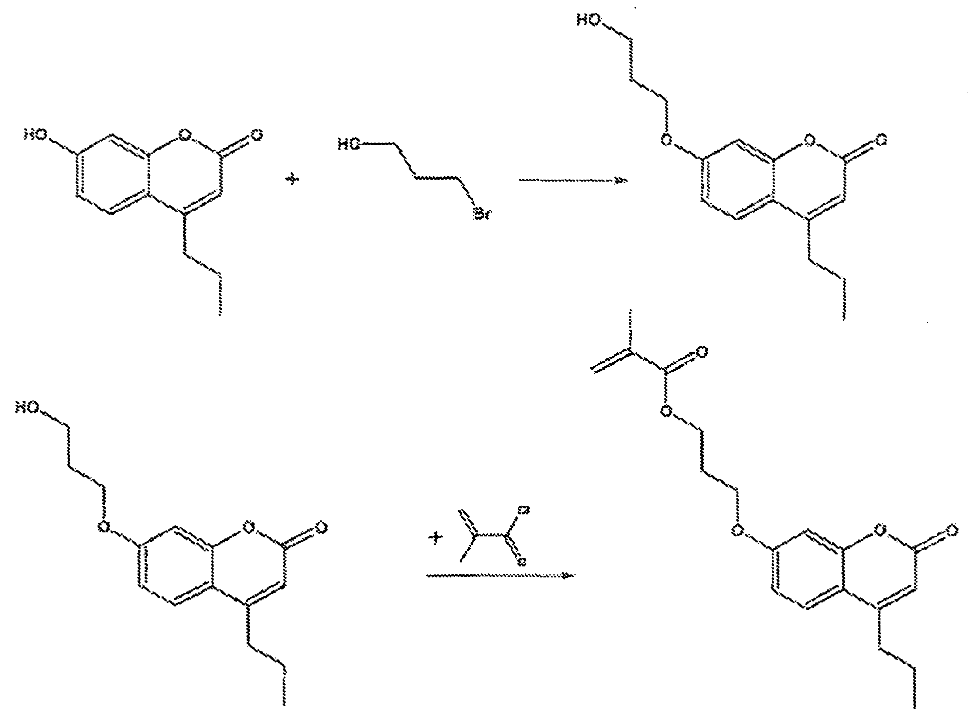

### Beispiel 5:

n = 2, m = 1, X = O, R2 = C₃H₆, Y = O, R1 = Acryl- bzw. Methacrylrest, R3 = H, R4 = H, R5 = H der allgemeinen Formel I.

Auch ein trifunktionaler UV-Absorber lässt sich auf einfachem synthetischem Wege herstellen. Ausgehend vom 4,5,7-Trihydroxycumarin erhält man nach der Alkoxylierung mit 3-Brom-1-propanol und nachfolgender Acrylierung bzw. Methacrylierung einen UV-Absorber mit drei Ankergruppen.

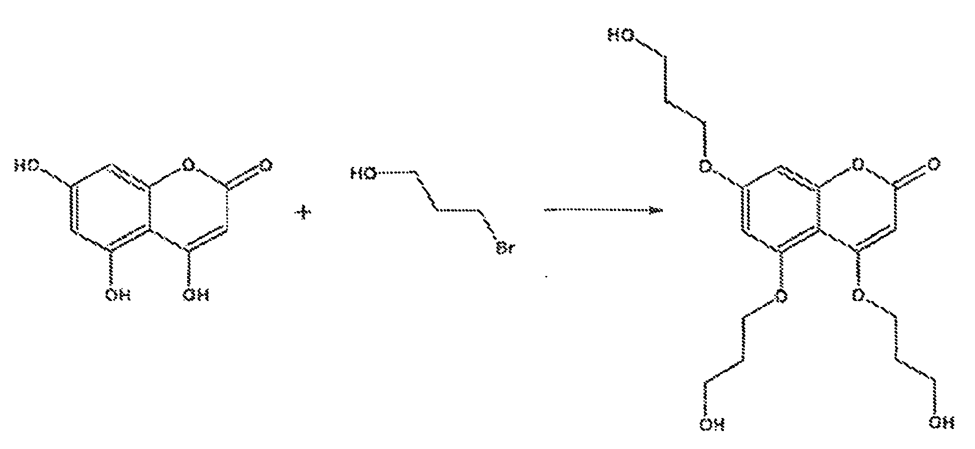

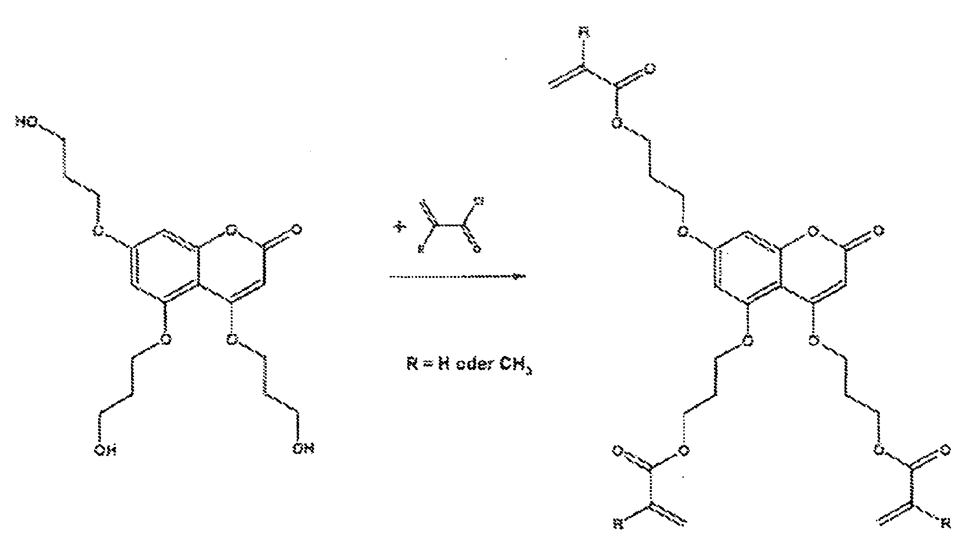

### Violett-Absorber

Geeignete Violett-Absorber der erfindungsgemäßen ophthalmologischen Zusammensetzung sind Verbindungen der folgenden Strukturen: 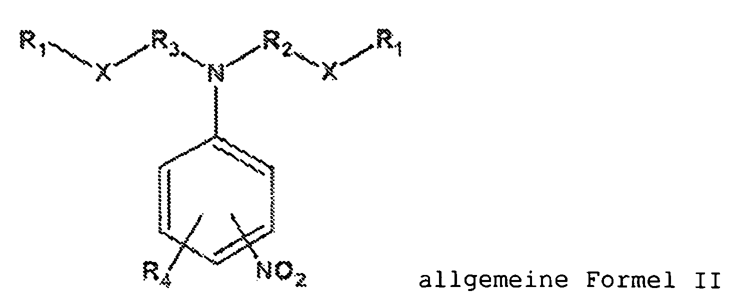 X = 0, S, NH, NR (R ist ein organischer verzweigter oder unverzweigter Alkyl- oder Aryl-Substituenten (oder Kombinationen von beiden) mit bis zu 30 Atomen ausgewählt aus C, H, Si, O, N, P, S, F, Cl, Br)
R1 = Acryl- oder Methacrylrest 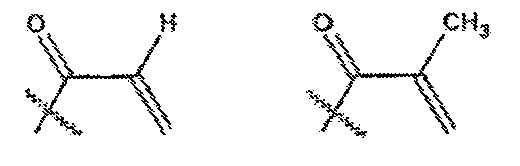 R2 = Organische verzweigte und unverzweigte Alkyl- oder Arylspacergruppe (oder Kombination aus beiden) mit bis zu 30 Atomen, ausgewählt aus: C, H, Si, O, N, P, S, Cl, Br, F
R3 = organische verzweigte und unverzweigte Alkyl- oder Arylspacergruppe (oder Kombination aus beiden) mit bis zu 30 Atomen, ausgewählt aus: C, H, Si, O, N, P, S, Cl, Br, F
R4 = H oder organische verzweigte und unverzweigte Alkylgruppe mit bis zu 30 Atomen, ausgewählt aus: C, H, Si, O, N, P, S, Cl, Br, F oder weitere Nitrogruppe, Alkoxygruppe oder Nitrilgruppe

Beispiele entsprechender Strukturen (alle Stereoisomere oder racemische Gemische sind beinhaltet) sind:

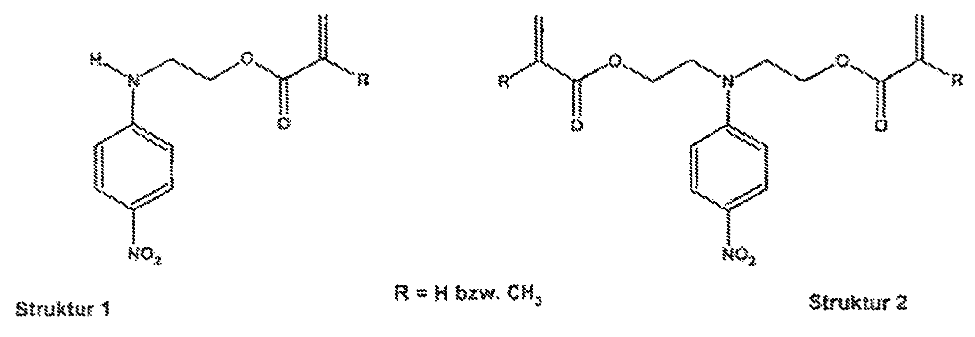

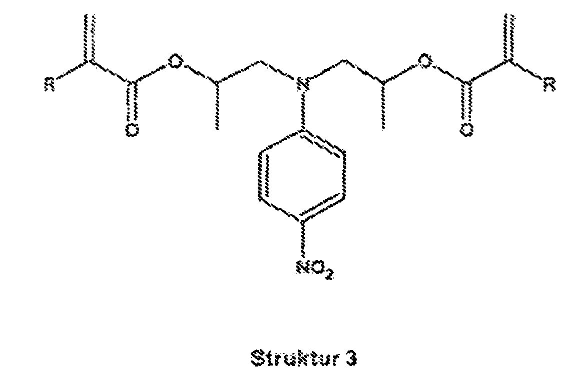

Ferner sind geeignete Violett-Absorber Stereoisomere oder racemische Gemische von Verbindungen folgender Strukturen: 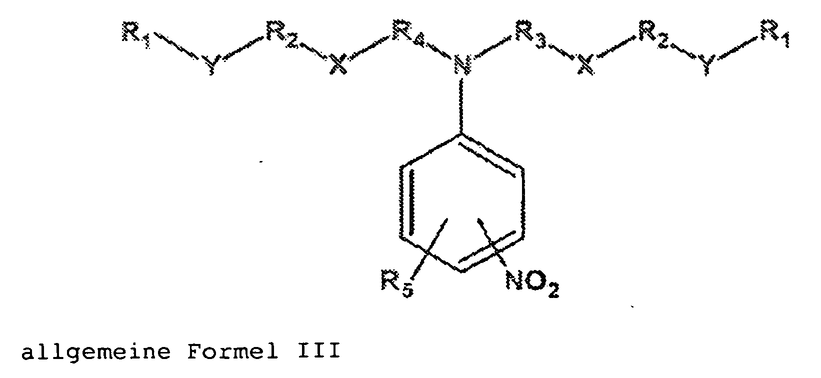 X = 0, S, NH, NR (R ist ein organischer verzweigter oder unverzweigter Alkyl- oder Aryl-Substituent (oder Kombinationen von beiden) mit bis zu 30 Atomen ausgewählt aus C, H, Si, O, N, P, S, F, Cl, Br)
Y = O, S, NH, NR (R ist ein organischer verzweigter oder unverzweigter Alkyl- oder Aryl-Substituent (oder Kombinationen von beiden) mit bis zu 30 Atomen ausgewählt aus C, H, Si, O, N, P, S, F, C1, Br)
R1 = Acryl- oder Methacrylrest
R2 = Organische verzweigte und unverzweigte Alkyl- oder Arylspacergruppe (oder Kombination aus beiden) mit bis zu 30 Atomen, ausgewählt aus: C, H, Si, O, N, P, S, Cl, Br, F
R3 = Organische verzweigte und unverzweigte Alkyl- oder Arylspacergruppe (oder Kombination aus beiden) mit bis zu 30 Atomen, ausgewählt aus: C, H, Si, O, N, P, S, Cl, Br, F
R4 = organische verzweigte und unverzweigte Alkyl- oder Arylspacergruppe (oder Kombination aus beiden) mit bis zu 30 Atomen, ausgewählt aus: C, H, Si, O, N, P, S, Cl, Br, F
R5 = H oder organische verzweigte und unverzweigte Alkylgruppe mit bis zu 30 Atomen, ausgewählt aus: C, H, Si, O, N, P, S, Cl, Br, F oder weitere Nitrogruppe, Alkoxygruppe oder Nitrilgruppe

Beispiele entsprechender Strukturen (alle Stereoisomere oder racemische Gemische sind beinhaltet) sind:

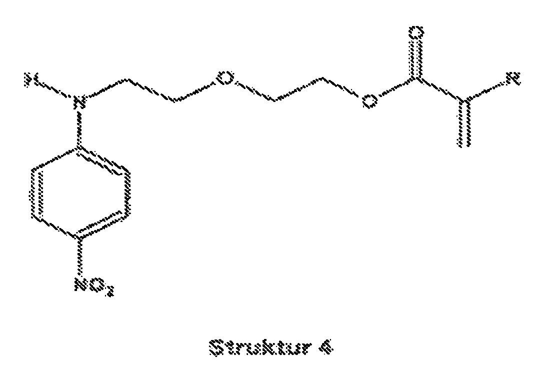

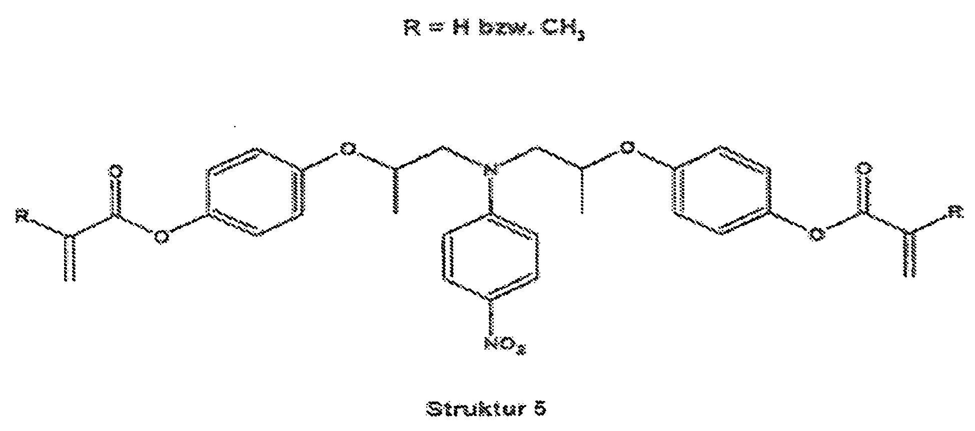

Ein bevorzugter Farbstoff für den Violett-Absorber der erfindungsgemäßen ophthalmologischen Zusammensetzung ist:
N, N-Di-2'-ethylmethacrylat-4-nitroanilin
mit der Struktur:

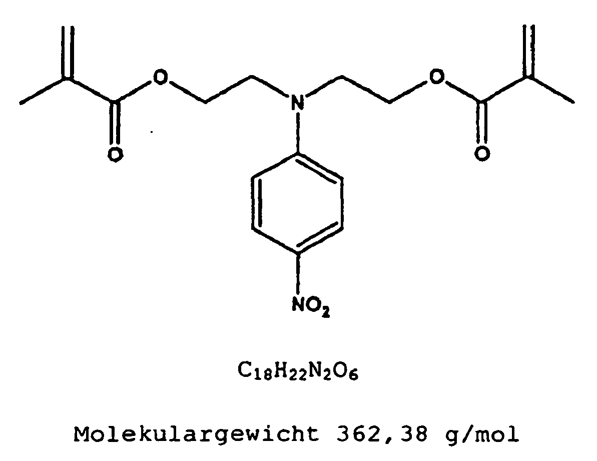

Die Herstellung dieser Verbindung erfolgt in zwei Schriten (gemäß EP 0321891 A2), wobei beide Edukte, sowohl das 4-Fluornitrobenzol, als auch das Diethanolamin kommerziell erhältlich sind:

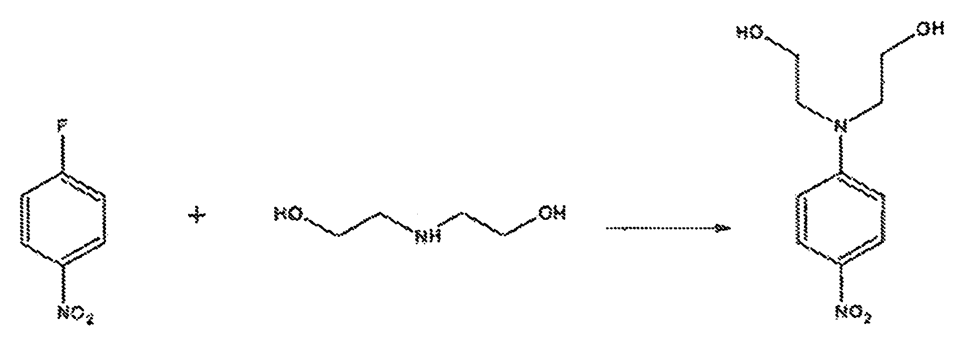

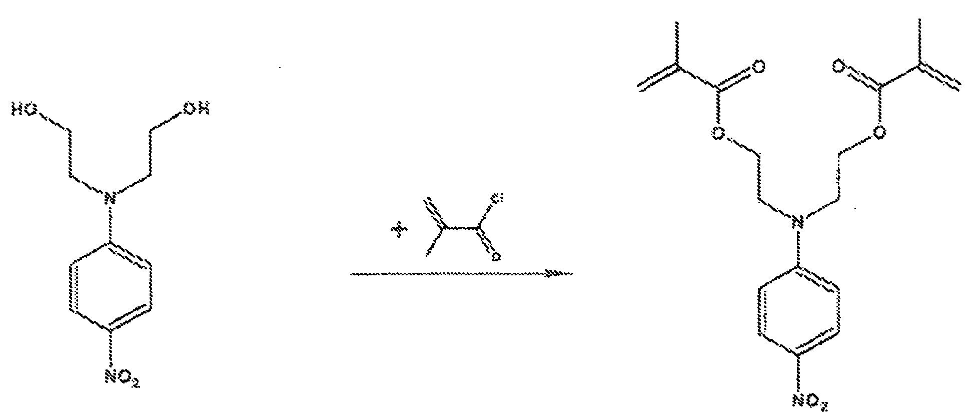

Die Methacryl- Reste dienen zur kovalenten Einbindung des Violett-Filters in das Copolymer bzw. ein Trägermaterial, insbesondere Linsenmaterial auf Acrylat-Basis. Aufgrund der Bifunktionalität verläuft der Einbau quantitativ und somit deutlich effektiver als bei den am Markt erhältlichen monofunktionalen Violett-Filtern.

Weitere Ausführungsbeispiele für den Violett-Absorber sind ebenfalls Verbindungen, bei denen ein Nitroanilin Grundkörper über verschiedene Spacer mit einem oder mehreren Acryl- bzw. Methacryl-Resten verbunden ist.

### Ausführungsbeispiele Violett-Absorber

### Beispiel 1:

R2 und R3 = -CH₂-CH(CH₃)-, X = O, R1 = Acryl- oder Methacrylrest, R4 = H der allgemeinen Formel II.

Ein weiteres Ausführungsbeispiel für einen gelben Chromophor/Violett-Filter ist das N,N-Di-2'-isopropylmethacrylat-4-nitroanilin. Auch dieses kann auf einfachem synthetischem Wege analog dem Diethylmethacrylat-4-nitroanilin in einer 2-stufigen Reaktion hergestellt werden. Das dazu benötigte Diisopropanolamin ist ebenfalls kommerziell verfügbar. Auf diese Weise kann eine Verbindung hergestellt werden, die sich vom bevorzugten Filter um nur jeweils eine CH₃-Gruppe in der Seitenkette unterscheidet. Durch den positiven induktiven Effekt der Methylgruppen absorbiert dieser Chromophor geringfügig zu längeren Wellenlängen verschoben.

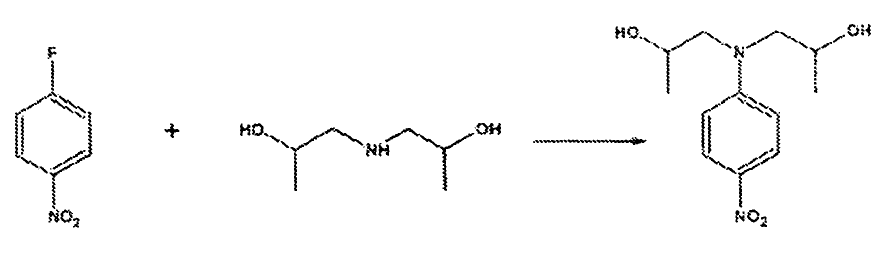

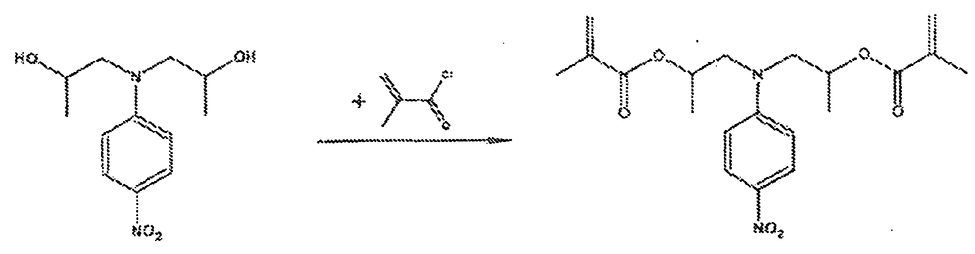

### Beispiel 2:

R2 und R3 = C₂H₄, X = NR, R1 = Acryl- oder Methacrylrest, R4 = H der allgemeinen Formel II.

Bei diesem Beispiel wird N,N-Dihydroxyethyl-4-nitroanilin durch eine einfache synthetische Methode in das Diamino-Derivat umgesetzt. Dieses Diamin kann durch eine Reaktion mit Acrylsäurechlorid in das Diamid umgewandelt werden. Die Struktur des Chromophors bleibt unverändert und ist durch zwei Methyleneinheiten vom Acryl-Amid getrennt.

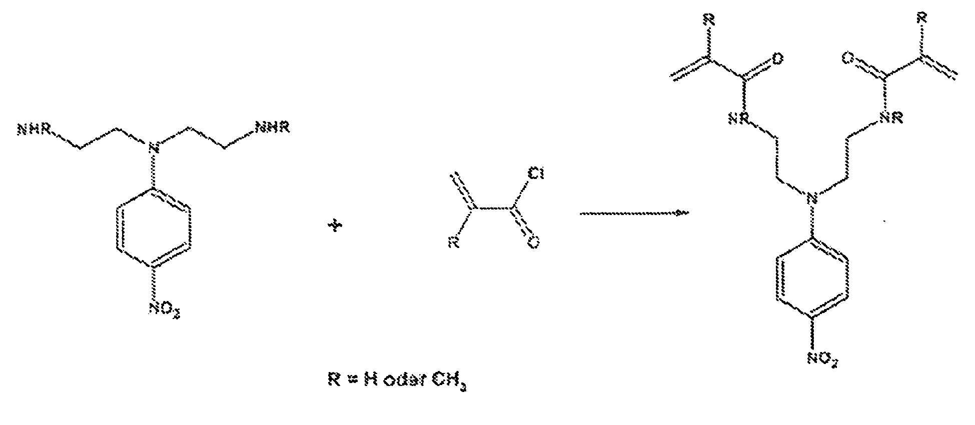

### Beispiel 3:

R3 und R4 = C₂H₄, X = O, R2 = -CH₂-CH(OH)-CH₂-, Y = O, R1 = Acryl- oder Methacrylrest, R5 = H der allgemeinen Formel III.

Setzt man N,N-Dihydroxyethyl-4-nitroanilin nicht mit Acrylsäure bzw. Methacrylsäurechlorid, sondern mit kommerziell erhältlichem Methacrylsäureglycidylester um, so erhält man in einem einzigen Reaktionsschritt einen weiteren Violett-Filter, in dem der Chromophor durch aliphatische Ketten von den Methacrylatresten getrennt ist.

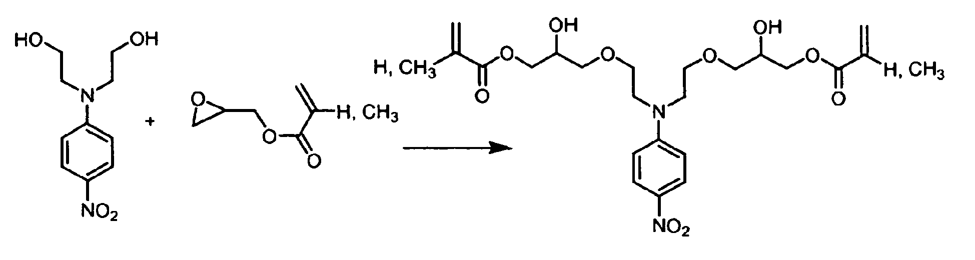

### Beispiel 4:

R2 und R3 = C₂H₄, X = NR, R1 = Acryl- oder Methacrylrest, R4 = CH₃ der allgemeinen Formel II.

Hier ist R4 eine Methylgruppe, die einen schwachen induktiven Effekt (+I-Effekt) besitzt. Das Einbringen einer zusätzlichen Methylgruppe in den zuvor beschriebenen bevorzugten Violett-Filter ist synthetisch ohne Probleme zu bewältigen und verändert die spektralen Eigenschaften des Chromophors nur in geringem Maße. Setzt man Diethanolamin nicht mit 4-Fluornitrobenzen, sondern mit dem ebenfalls kommerziell erhältlichen 2-Fluor-5-nitrotoluol um, so wird ein Nitroanilin erzeugt, das sich vom bevorzugten Violett-Absorber um gerade eine zusätzliche Methylgruppe am Anilin-Ring unterscheidet. Durch Veresterung mit Acryloylchlorid oder Methacryloylchlorid wird so ein weiterer Chromophor mit den gewünschten spektralen Eigenschaften erhalten.

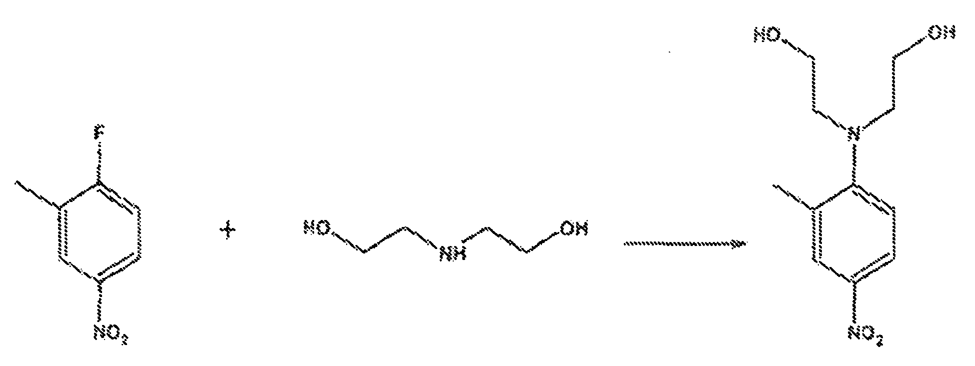

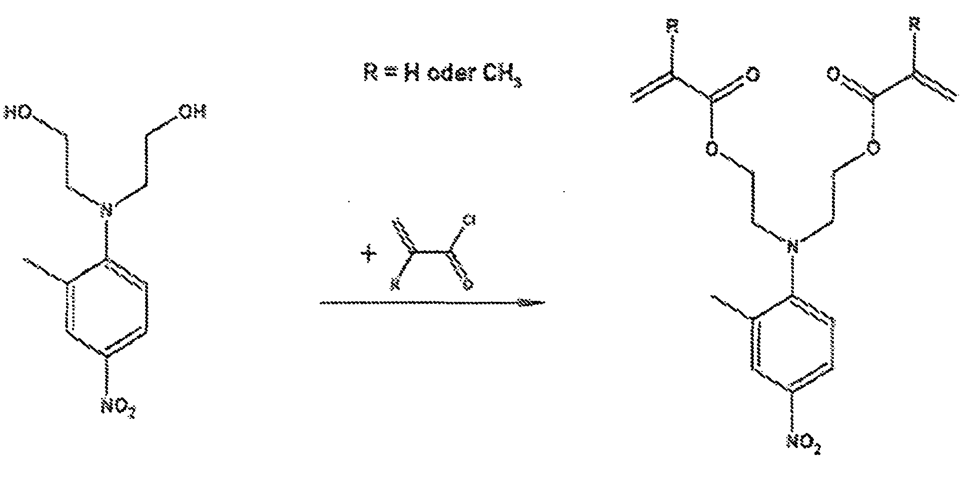

Als biokompatibles Trägermaterial sind Acrylate, insbesondere mit einem Wassergehalt von 1% bis 30%, für die ophthalmologische Zusammensetzung geeignet. In dem Copolymer bzw. in diesem Trägermaterial sind der UV-Absorber bzw. der Violett-Absorber kovalent eingebunden. Der UV-Absorber ist vorzugsweise in einem Konzentrationsbereich von 0,5 % bis 1,0 % enthalten. Wenn die ophthalmologische Zusammensetzung für eine IOL verwendet wird, hängt die jeweilige Konzentration des UV-Absorbers vom jeweiligen Scheitelbrechwert (Dioptrie) der Linse ab. Der Violett-Absorber ist ebenfalls kovalent im Acrylat-Trägermaterial bzw. im Copolymer gebunden. Er kann in einem Konzentrationsbereich von 0,03% bis 0,16% vorliegen. Auch hier hängt bei der Verwendung der ophthalmologischen Zusammensetzung für eine IOL die Konzentration des Violett-Absorbers direkt von der Dioptrie der Linse ab.

Die Gefahr des Auswaschens der Absorber aus der Trägermatrix besteht nicht, da sowohl der erfindungsgemäße UV-Absorber als auch der Violett-Filter bedingt dadurch, dass sie zwei polymerisationsfähige Endgruppen tragen, quantitativ in das Linsenmaterial einbauen.

Geeignete biokompatible Trägermaterialien für den UV-Absorber bzw. den Violett-Absorber sind beispielsweise Hydroxyethylmethacrylat (HEMA), Methylmethacrylat (MMA), Ethoxyethylmethacrylat (EOEMA), Ethoxyethoxyethylacrylat (EEEA), Tetrahydrofufurylmethacrylat (THFMA), Tetrahydrofufurylacrylat (THFA), 2-Hydroxypropylmethacrylat (HPMA), 2-Hydroxypropylacrylat (HPA), 2-Hydroxyethylacrylamid, 2-Hydroxyethylmethacrylamid, Methoxyethylmethacrylat (MOEMA) und Methoxyethylacrylat (MOEA). Aus den zuvor genannten Stoffen können Copolymerisate, evtl. unter Verwendung eines Quervemetzers, hergestellt und als Trägermaterial verwendet werden. Die prozentuale Zusammensetzung der Monomere ist in einem weiten Bereich variabel. Die Trägermaterialien können hydrophil mit einem Wassergehalt von beispielsweise 1% bis 30% oder hydrophob eingestellt werden. Begrenzender Faktor bei hydrophoben, wasserfreien Polymeren ist der Glaspunkt. Dieser kann im Bereich zwischen 0°C und 11°C liegen. Darüber hinaus ist wichtig, dass hydrophile Polymere nach der Quellung eine ausreichende Flexibilität aufweisen.

Ein Ausführungsbeispiel der ophthalmologischen Zusammensetzung ist folgendes mit quantitativen Zusammensetzungen in Gewichts %:

### Ausführungsbeispiel Trägermaterialien

### Beispiel 1 (hydrophil)

HEMA (Hydroxyethylmethacrylat) 50 - 85 Gew.-%
EOEMA (Ethoxyethylmethacrylat) 30 - 40 Gew.-%
THFMA (Tetrahydrofufurylmethacrylat) 5 - 20 Gew.-%
EGDMA (Ethylenglykoldimethacrylat) 0 - 0,7 Gew.-%
UV-Absorber 0,1- 1,0 Gew.-%
Violett-Absorber 0,03 - 0,16 Gew.-%

Zur Synthese der jeweiligen Linsenmaterialien werden in ein Becherglas nacheinander zunächst die Monomere eingewogen und solange gerührt, bis eine homogene Lösung entstanden ist. Danach wird zunächst der Vernetzer und nachfolgend der Violett- sowie der UV-Absorber zugegeben. Unter leichtem Erwärmen wird wiederum so lange nachgerührt bis eine homogene Lösung erhalten wird.

Die sich jeweils ergebende Mischung wird mit einem geeigneten Initiator versetzt und in die Polymerisationsformen überführt (z.B. Näpfe, Stangen- oder Plattformen). Die Polymerisation wird durch Erhitzen eingeleitet (60°C für 12-16 h). Nach dem Erkalten werden die Polymerisate entnommen, ggf. im Trockenschrank nachgehärtet und durch Drehen und Fräsen auf die gewünschte Rohlingsgröße gebracht (z.B. 3 mm Stärke, 12,7 mm Durchmesser).

Transmissionsmessungen zeigen, dass mit Hilfe der erfindungsgemäßen ophthalmologischen Zusammensetzung nicht nur der UV-Anteil (<400 nm), sondern auch der gesamte violette Lichtanteil (400 nm bis 430 nm) absorbiert wird. Auf dem Markt befindliche ophthalmologische Zusammensetzungen weisen im violetten Bereich eine hohe Lichtdurchlässigkeit mit bis zu einem Drittel Transmission auf. Die erfindungsgemäße Zusammensetzung zeigt bei 430 nm lediglich eine Transmission von unter 3 %.

Im blauen Lichtbereich hat die erfindungsgemäße Zusammensetzung beispielsweise bei 460 nm eine Lichtdurchlässigkeit von über 70 %, während die bekannten Linsen hier nur 50 -60 % Durchlässigkeit aufweisen.

Die ophthalmologische Zusammensetzung eignet sich insbesondere für Sehhilfen, wie Brillen, Kontaktlinsen und Augenimplantate. Insbesondere eignet sich die ophthalmologische Zusammensetzung für Intraokularlinsen.

Durch die ophthalmologische Zusammensetzung wird ein hohes Maß an Photoprotektion bei gleichzeitiger maximaler Photorezeption gewährleistet. Diese Zusammensetzung muss also im wesentlichen den gesamten ultravioletten Spektralbereich und den violetten Lichtanteil des sichtbaren Spektrums absorbieren, dabei aber gleichzeitig die vollständige Transmission von blauem Licht, insbesondere des Wellenlängenbereichs zwischen 450 nm und 500 nm, ermöglichen.

Weitere Merkmale der Erfindung ergeben sich aus den Ansprüchen und der Beschreibung. Die vorstehend in der Beschreibung genannten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Auch die ophthalmologische Zusammensetzung oder eine vorteilhafte Ausführung davon kann eine Ausführung des erfindungsgemäßen Copolymers aufweisen.

## Patentansprüche

1. Ophthalmische Linse umfassend ein Copolymer, welches:
a. bezogen auf das Gesamtgewicht des Copolymers 20 bis 95 Gewichtsprozent Struktureinheiten, stammend aus wenigstens einem hydrophilen Monomer, und
b. bezogen auf das Gesamtgewicht des Copolymers 5 bis 80 Gewichtsprozent Struktureinheiten, stammend aus wenigstens einem Monomer gemäß der allgemeinen Formel I 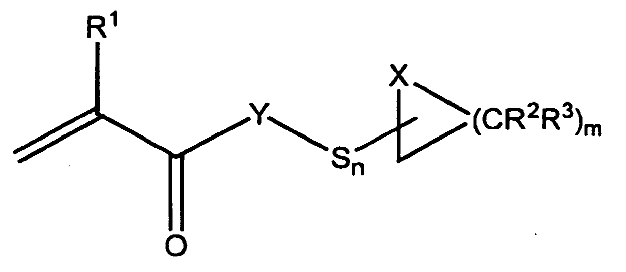 umfasst, wobei
R¹, R² und R³ jeweils unabhängig voneinander Wasserstoff oder Alkyl- bedeuten,
Y: O oder NR⁴ mit R⁴ ausgewählt aus Wasserstoff oder Alkyl- bedeutet,
X: O, S, SO oder SO₂ bedeutet,
S: eine Struktureinheit ausgewählt aus CHR⁵ oder (CHR-⁵CHR⁵O)ᵢCH₂ bedeutet, wobei alle R⁵ jeweils unabhängig voneinander Wasserstoff oder Alkyl- bedeuten, n und i unabhängig voneinander eine ganze Zahl zwischen 1 und 10 und
m eine ganze Zahl zwischen 2 und 6 bedeuten,
und wobei das Copolymer bezogen auf das Gesamtgewicht des Copolymeren nach der Quellung in Wasser einen Wassergehalt von 1 bis 59 Gewichtsprozent aufweist.

2. Ophthalmische Linse nach Anspruch 1, **dadurch gekennzeichnet, dass** das Monomer b) folgende allgemeine Struktur 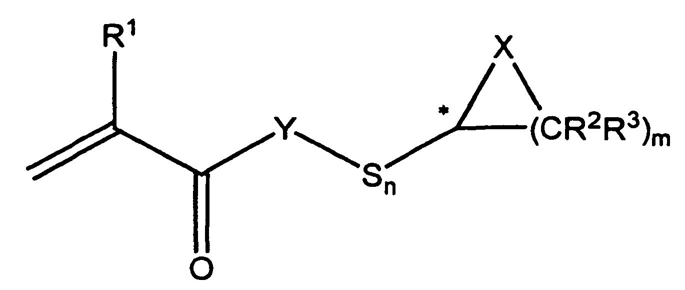 aufweist.

3. Ophthalmische Linse nach Anspruch 1 oder 2, **dadurch gekennzeichnet, dass** bezogen auf das Gesamtgewicht des Copolymers 21 bis 80 Gewichtsprozent Struktureinheiten aus wenigstens einem Monomer gemäß der allgemeinen Formel I stammen.

4. Ophthalmische Linse nach einem der Ansprüche 1 bis 3, **dadurch gekennzeichnet, dass** das hydrophile Monomer a) ein Monomer der allgemeinen Formel II 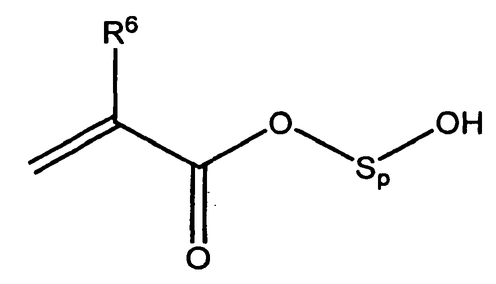 ist, wobei
S: eine Struktureinheit ausgewählt aus CHR⁷ oder (CHR⁷CHR⁷O)ₖCH₂ bedeutet, wobei alle R⁷ jeweils unabhängig voneinander Wasserstoff oder Alkyl- bedeuten und
p und k unabhängig voneinander eine ganze Zahl zwischen 1 und 10 bedeuten.

5. Ophthalmische Linse nach einem der Ansprüche 1 bis 4, **dadurch gekennzeichnet, dass** das hydrophile Monomer Hydroxyethylmethacrylat (HEMA) und/oder Hydroxypropylmethacrylat (HPMA) und/oder Glycerolmonomethacrylat ist.

6. Ophthalmische Linse nach einem der vorangehenden Ansprüche, **dadurch gekennzeichnet, dass** das Monomer der allgemeinen Formel I Tetrahydrofurfurylmethacrylat (THFMA) ist.

7. Ophthalmische Linse nach einem der vorangehenden Ansprüche, **dadurch gekennzeichnet, dass** das Copolymer einen UV-Absorber und/oder einen Violett-Absorber (Gelbfarbstoff enthält.

8. Ophthalmische Linse nach einem der vorangehenden Ansprüche, **dadurch gekennzeichnet, dass** das Copolymer einen oder mehrere Vernetzer enthält.

9. Ophthalmische Linse nach Anspruch 8, **dadurch gekennzeichnet, dass** wenigstens ein Teil des Vernetzers oder der Vernetzer ein Violett-Absorber oder ein UV-Absorber ist.

10. Ophthalmische Linse nach einem der vorangehenden Ansprüche, **dadurch gekennzeichnet, dass** das Copolymer einen Brechungsindex von wenigstens 1,3 aufweist.

11. Ophthalmische Linse nach einem der vorangehenden Ansprüche, wobei die Linse eine Intraokularlinse und/oder ein ophthalmisches Implantat ist.

12. Ophthalmische Linse nach einem der vorangehenden Ansprüche, **dadurch gekennzeichnet, dass** die ophthalmische Linse einteilig oder mehrteilig ist.

13. Ophthalmische Linse nach einem der vorangehenden Ansprüche, **dadurch gekennzeichnet, dass** die ophthalmische Linse faltbar ist.

14. Verwendung eines Copolymers zur Herstellung einer ophthalmischen Linse, einer Intraokularlinse und/oder eines ophthalmischen Implantates, wobei das Copolymer:
a. bezogen auf das Gesamtgewicht des Copolymers 20 bis 95 Gewichtsprozent Struktureinheiten, stammend aus wenigstens einem hydrophilen Monomer, und
b. bezogen auf das Gesamtgewicht des Copolymers 5 bis 80 Gewichtsprozent Struktureinheiten, stammend aus wenigstens einem Monomer gemäß der allgemeinen Formel I 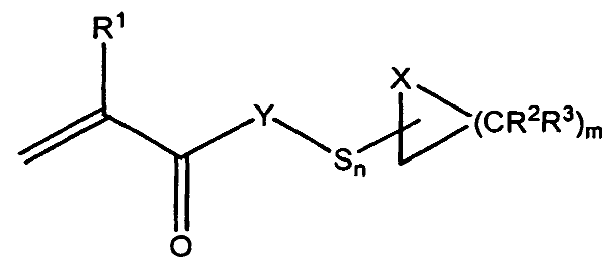 umfasst, wobei
R¹, R² und R³ jeweils unabhängig voneinander Wasserstoff oder Alkyl- bedeuten,
Y: O oder NR⁴ mit R⁴ ausgewählt aus Wasserstoff oder Alkyl- bedeutet,
X: O, S, SO oder SO₂ bedeutet,
S: eine Struktureinheit ausgewählt aus CHR⁵ oder (CHR⁵CHR⁵O)ᵢCH₂ bedeutet, wobei alle R⁵ jeweils unabhängig voneinander Wasserstoff oder Alkyl- bedeuten, n und i unabhängig voneinander eine ganze Zahl zwischen 1 und 10 und
m eine ganze Zahl zwischen 2 und 6 bedeuten,
und wobei das Copolymer bezogen auf das Gesamtgewicht des Copolymeren nach der Quellung in Wasser einen Wassergehalt von 1 bis 59 Gewichtsprozent aufweist.

## Claims

1. Ophthalmic lens comprising a copolymer, and said copolymer comprising:
a) 20 to 95 percent by weight, based on the total weight of the copolymer, of structural units derived from at least one hydrophilic monomer, and
b) 5 to 80 percent by weight, based on the total weight of the copolymer, of structural units derived from at least one monomer according to the general formula I 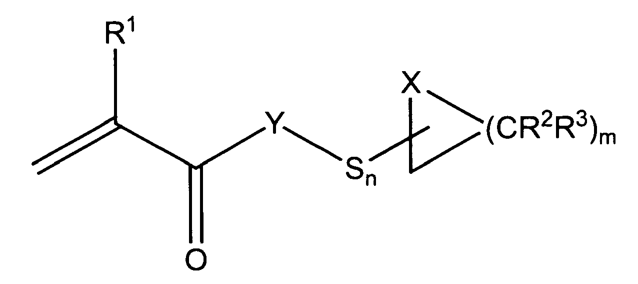 wherein
R¹, R² and R³ each independently of each other denote hydrogen or alkyl,
Y: denotes O or NR⁴ with R⁴ selected from hydrogen or alkyl,
X: denotes O, S, SO or SO₂,
S: denotes a structural unit selected from CHR⁵ or (CHR⁵CHR⁵O)ᵢCH₂, wherein all of the R⁵ each independently of each other denote hydrogen or alkyl,
n and i independently of each other denote an integer between 1 and 10 and m denotes an integer between 2 and 6,
and wherein the copolymer has a water content from 1 to 59 percent by weight based on the total weight of the copolymer.

2. Ophthalmic lens according to claim 1, **characterized in that** the monomer b) has the following general structure

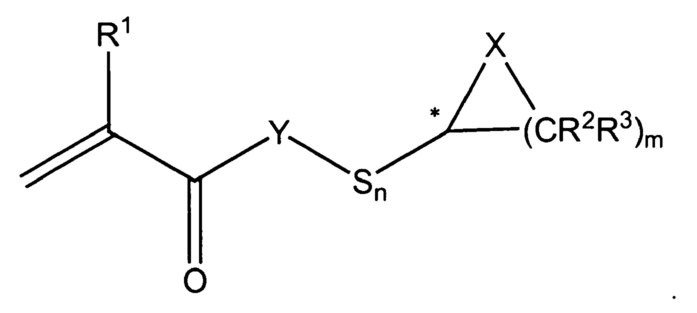

3. Ophthalmic lens according to claim 1 or 2, **characterized in that** 21 to 80 percent by weight, based on the total weight of the copolymer, of structural units are derived from at least one monomer according to the general formula I.

4. Ophthalmic lens according to any one of claims 1 to 3, **characterized in that** the hydrophilic monomer a) is a monomer of the general formula II 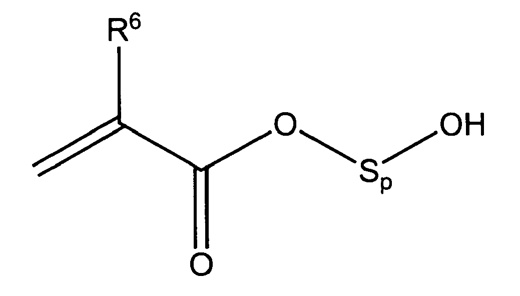 wherein
S: denotes a structural unit selected from CHR⁷ or (CHR⁷CHR⁷O)ₖCH₂, wherein all of the R⁷ each independently of each other denote hydrogen or alkyl and
p and k independently of each other denote an integer between 1 and 10.

5. Ophthalmic lens according to any one of claims 1 to 4, **characterized in that** the hydrophilic monomer is hydroxyethyl methacrylate (HEMA) and/or hydroxypropyl methacrylate (HPMA) and/or glycerol monomethacrylate.

6. Ophthalmic lens according to anyone of the preceding claims, **characterized in that** the monomer of the general formula I is tetrahydrofurfuryl methacrylate (THFMA).

7. Ophthalmic lens according to anyone of the preceding claims, **characterized in that** the copolymer contains an UV absorber and/or a violet absorber (yellow dye).

8. Ophthalmic lens according to anyone of the preceding claims, **characterized in that** the copolymer contains one or more cross-linkers.

9. Ophthalmic lens according to claim 8, **characterized in that** at least a portion of the cross-linker or the cross-linkers is a violet absorber or an UV absorber.

10. Ophthalmic lens according to anyone of the preceding claims, **characterized in that** the copolymer has a refractive index of at least 1.3.

11. Ophthalmic lens according to anyone of the preceding claims, wherein the lens is an intraocular lens and/or an ophthalmic implant.

12. Ophthalmic lens according to anyone of the preceding claims, **characterized in that** the ophthalmic lens is one-piece or multi-piece.

13. Ophthalmic lens according to anyone of the preceding claims, **characterized in that** the ophthalmic lens is foldable.

14. Use of a copolymer for the production of an ophthalmic lens, an intraocular lens and/or an ophthalmic implant, wherein the copolymer comprising:
a) 20 to 95 percent by weight, based on the total weight of the copolymer, of structural units derived from at least one hydrophilic monomer, and
b) 5 to 80 percent by weight, based on the total weight of the copolymer, of structural units derived from at least one monomer according to the general formula I 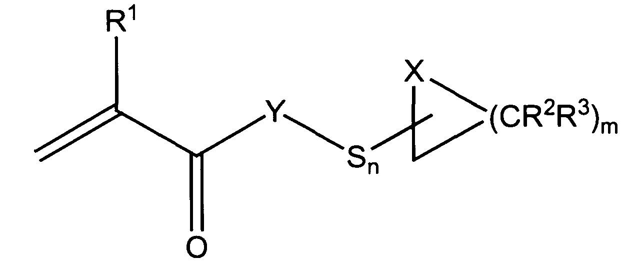 wherein
R¹, R² and R³ each independently of each other denote hydrogen or alkyl-,
Y: denotes O or NR⁴ with R⁴ selected from hydrogen or alkyl-,
X: denotes O, S, SO or SO₂,
S: denotes a structural unit selected from CHR⁵ or (CHR⁵CHR⁵O)ᵢCH₂, wherein all of the R⁵ each independently of each other denote hydrogen or alkyl-,
n and i independently of each other denote an integer between 1 and 10 and
m denotes an integer between 2 and 6,
and wherein the copolymer has a water content from 1 to 59 percent by weight based on the total weight of the copolymer.

## Revendications

1. Lentille ophtalmique comprenant un copolymère, lequel comprend :
a. par rapport au poids total du copolymère, 20 à 95 pour cent en poids d'unités structurales, provenant d'au moins un monomère hydrophile et
b. par rapport au poids total du copolymère, 5 à 80 pour cent en poids d'unités structurales, provenant d'au moins un monomère, conformément à la formule générale 1, 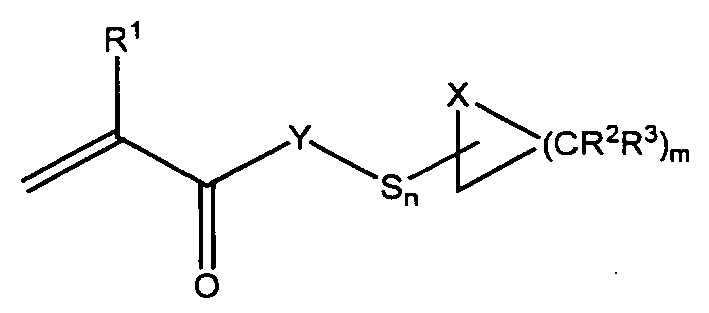 moyennant quoi
R¹, R² et R³ signifient respectivement, indépendamment les uns des autres, de l'hydrogène ou de l'alkyle,
Y : signifie O ou NR⁴ avec R⁴, sélectionné à partir d'hydrogène ou d'alkyle,
X : signifie O, S, SO ou SO₂,
S : signifie une unité structurale, sélectionnée à partir de CHR⁵ ou de (CHR⁵CHR⁵O)ᵢCH₂, moyennant quoi tous les R⁵ signifient respectivement, indépendamment les uns des autres, de l'hydrogène ou de l'alkyle,
n et i signifient, indépendamment les uns des autres, un nombre entier entre 1 et 10 et
m signifie un nombre entier entre 2 et 6
et moyennant quoi le copolymère présente, par rapport au poids total du copolymère, après le gonflement dans l'eau, une teneur en eau de 1 à 59 pour cent en poids.

2. Lentille ophtalmique suivant la revendication 1, **caractérisée en ce que** le monomère b) présente la structure générale suivante :

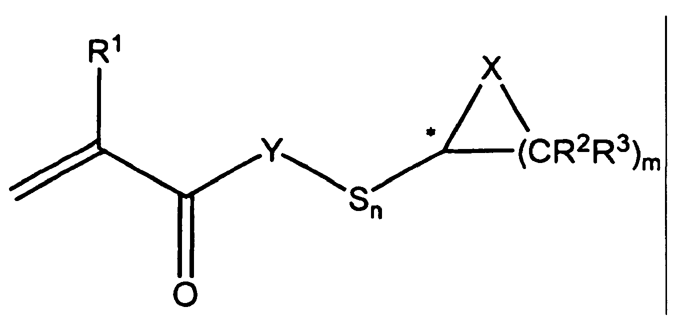

3. Lentille ophtalmique suivant la revendication 1 ou 2, **caractérisée en ce que**, par rapport au poids total du copolymère, 21 à 80 pour cent en poids d'unités structurales proviennent d'au moins un monomère, conformément à la formule générale I.

4. Lentille ophtalmique suivant l'une des revendications 1 à 3, **caractérisée en ce que** le monomère hydrophile a) est un monomère de la formule générale II, 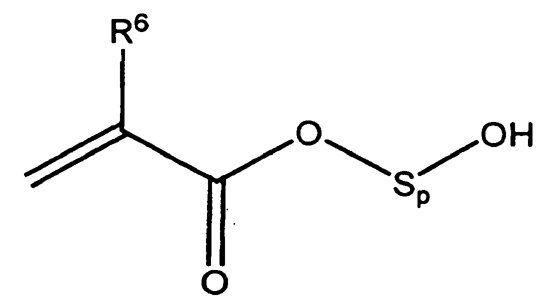 moyennant quoi
S : signifie une unité structurale, sélectionnée à partir de CHR⁷ ou (CHR⁷CHR⁷O)ₖCH₂, moyennant quoi tous les R⁷ signifient respectivement, indépendamment les uns des autres, de l'hydrogène ou de l'alkyle et
p et k signifient, indépendamment l'un de l'autre, un nombre entier entre 1 et 10.

5. Lentille ophtalmique suivant l'une des revendications 1 à 4, **caractérisée en ce que** le monomère hydrophile est du méthacrylate d'hydroxyéthyle (HEMA) et / ou du méthacrylate d'hydroxypropyle (HPMA) et / ou du monométhacrylate de glycérol.

6. Lentille ophtalmique suivant l'une des revendications précédentes, **caractérisée en ce que** le monomère de la formule générale I est du méthacrylate de tétrahydrofurfuryle (THFMA).

7. Lentille ophtalmique suivant l'une des revendications précédentes, **caractérisée en ce que** le copolymère contient un absorbeur d'UV et / ou un absorbeur de violets (jaune).

8. Lentille ophtalmique suivant l'une des revendications précédentes, **caractérisée en ce que** le copolymère contient un ou plusieurs agents de réticulation.

9. Lentille ophtalmique suivant la revendication 8, **caractérisée en ce qu'**au moins une partie de l'agent de réticulation ou des agents de réticulation est un absorbeur de violets ou un absorbeur d'UV.

10. Lentille ophtalmique suivant l'une des revendications précédentes, **caractérisée en ce que** le copolymère présente un indice de réfraction d'au moins 1,3.

11. Lentille ophtalmique suivant l'une des revendications précédentes, moyennant quoi la lentille est une lentille intraoculaire et / ou un implant ophtalmique.

12. Lentille ophtalmique suivant l'une des revendications précédentes, **caractérisée en ce que** la lentille ophtalmique est composée d'une seule partie ou de plusieurs parties.

13. Lentille ophtalmique suivant l'une des revendications précédentes, **caractérisée en ce que** la lentille ophtalmique est pliable.

14. Utilisation d'un copolymère, destiné à la fabrication d'une lentille ophtalmique, d'une lentille intraoculaire et / ou d'un implant ophtalmique, moyennant quoi le copolymère comprend :
a. par rapport au poids total du copolymère, 20 à 95 pour cent en poids d'unités structurales, provenant d'au moins un monomère hydrophile et
b. par rapport au poids total du copolymère, 5 à 80 pour cent en poids d'unités structurales, provenant d'au moins un monomère, conformément à la formule générale I, 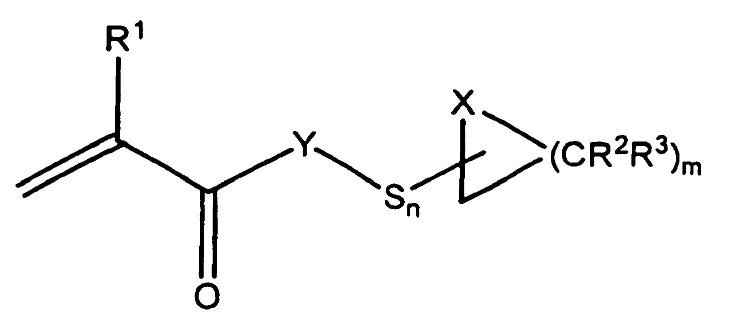 moyennant quoi
R¹, R² et R³ signifient respectivement, indépendamment les uns des autres, de l'hydrogène ou de l'alkyle,
Y : signifie O ou NR⁴ avec R⁴, sélectionné à partir d'hydrogène ou d'alkyle,
X : signifie O, S, SO ou SO₂,
S : signifie une unité structurale, sélectionnée à partir de CHR⁵ ou de (CHR⁵CHR⁵O)ᵢCH₂, moyennant quoi tous les R⁵ signifient respectivement, indépendamment les uns des autres, de l'hydrogène ou de l'alkyle,
n et i signifient, indépendamment les uns des autres, un nombre entier entre 1 et 10 et
m signifie un nombre entier entre 2 et 6
et moyennant quoi le copolymère présente, par rapport au poids total du copolymère, après le gonflement dans l'eau, une teneur en eau de 1 à 59 pour cent en poids.
